(19)

Europäisches
Patentamt
European
Patent Office
Office européen
des brevets

(11)  **EP 2 260 753 A1**

(12)  **EUROPEAN PATENT APPLICATION**
published in accordance with Art. 153(4) EPC

(43) Date of publication:
**15.12.2010 Bulletin 2010/50**

(51) Int Cl.:
**A61B 1/04** *(2006.01)*    **G03B 21/10** *(2006.01)*

(21) Application number: **08873544.4**

(22) Date of filing: **20.10.2008**

(86) International application number:
**PCT/JP2008/068973**

(87) International publication number:
**WO 2009/118938 (01.10.2009 Gazette 2009/40)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **25.03.2008 JP 2008079193**

(71) Applicants:
• **Panasonic Electric Works Co., Ltd**
**Kadoma-shi**
**Osaka 571-8686 (JP)**
• **Kyushu University,**
**National University Corporation**
**Higashi-ku**
**Fukuoka-shi**
**Fukuoka 812-8581 (JP)**

(72) Inventors:
• **YAMAMOTO, Atsuyuki**
**Kadoma-shi**
**Osaka 571-8686 (JP)**
• **HOSHINO, Hiroshi**
**Kadoma-shi**
**Osaka 571-8686 (JP)**
• **KAWAMURA, Ryo**
**Kadoma-shi**
**Osaka 571-8686 (JP)**
• **HASHIZUME, Makoto**
**Higashi-ku, Fukuoka-shi,**
**Fukuoka 812-8581 (JP)**
• **OHUCHIDA, Kenoki**
**Higashi-ku, Fukuoka-shi,**
**Fukuoka 812-8581 (JP)**

(74) Representative: **Appelt, Christian W.**
**Forrester & Boehmert**
**Pettenkoferstrasse 20-22**
**80336 München (DE)**

(54)  **ENDOSCOPE SYSTEM AND ENDOSCOPIC OPERATION TRAINING SYSTEM**

(57)    An endoscope system or an endoscopic operation training system includes: projectors 12 and 13 which project video light indicating a video taken by an endoscope device that allows at least a part thereof to be inserted into patient's body cavity; and a dome type screen 11 having a shape of a projection surface 11a that directs a concave surface toward an operator and assistants, in which the video light is projected onto the projection surface 11a. In a case where an axis that passes through a center of an opening surface thereof and is perpendicular to the opening surface is defined as a first axis, a point where the first axis and the projection surface 11a intersect each other is defined as a projection surface center, and an axis that connects the projection surface center and an edge portion of the projection surface 11a to each other is defined as a second axis, and a tangential line of the edge portion of the projection surface 11a is defined as a third axis, then an angle made by the first axis and the third axis is an angle at which it is possible to observe a whole of the video from the first viewpoint position, and an angle made by the first axis and the second axis is an axis at which it is possible to observe the projection surface center from the second viewpoint position.

FIG. 1

TO ENDOSCOPE DEVICE

EP 2 260 753 A1

**Description**

TECHNICAL FIELD

The present invention relates to an endoscope system that presents an affected area to an operator and the like in an endoscopic operation, and to an endoscopic operation training system that trains the operator and the like for a variety of tasks in the endoscopic operation.

BACKGROUND ART

Heretofore, an endoscopic surgical operation (hereinafter, referred to as an endoscopic operation) has been a low-invasive operation that brings, to a patient, a great deal of merits that an operation wound pain is small, that early ambulation and discharge are possible, and that an excellent advantage is also obtained in terms of beauty treatment. An endoscope system that realizes this endoscopic operation includes: an endoscope device that images an affected area; and a monitor that displays a video taken by the endoscope device, and displays a state of the affected area on the monitor. In this state, forceps inserted toward the affected area are manipulated, and the operation is implemented for the affected area. A video display apparatus usable for the endoscopic operation as described above is described, for example, in Patent Citation (Japanese Patent Laid-Open Publication No. 2008-15470) or the like.

DISCLOSURE OF THE INVENTION

At a site of the above-mentioned endoscopic operation, viewpoint positions of an operator and an assistant for a video are restrained owing to arrangement of a display screen, a bed and a variety of instruments. In the case where the viewpoint positions are restrained as described above, when the operator changes the viewpoint position, there are apprehensions that the operator cannot look at a center of the video, and that the operator cannot see the whole of the video, resulting in a possibility to cause a stress to the operator.

Further, in the endoscopic operation, it is necessary that a single video be seen by a person who manipulates a camera and other assistants as well as the operator who manipulates the forceps. In this case, it becomes important to allow visual recognition of a clear stereoscopic video from whichever angle the video concerned may be seen.

In this connection, the present invention has been proposed in consideration of the above-mentioned actual circumstances. It is an object of the present invention to provide an endoscope system and an endoscopic operation training system, which are capable of always presenting the clear stereoscopic video to the operator in the endoscopic operation.

The present invention is concerned with an endoscopic system that acquires a video of an imaging target in a patient's body cavity at an operation site where an operation instrument inserted into the patient's body cavity is manipulated by a first operator at a first viewpoint position.

The present invention includes: an endoscope device that is manipulated by a second operator located at a second viewpoint position, and takes a video of a patient's affected area by allowing at least a part thereof to be inserted into the patient's body cavity; video projecting means for projecting video light indicating the video taken by the endoscope device; video displaying means having a shape of a projection surface that directs a concave surface toward the first operator and the second operator, in which the video light is projected onto the projection surface by the video projecting means; and video signal processing means for performing, based on a positional relationship between at least the first viewpoint position and the video displaying means and on the shape of the projection surface, distortion correction processing for a video signal inputted to the video projecting means so that the video can be displayed without distortion on the projection surface when seen from the first viewpoint position.

In order to solve the above-mentioned problem, in the video displaying means in the present invention, in a case where an axis that passes through a center of an opening surface serving as the concave surface and is perpendicular to the opening surface is defined as a first axis, a point where the first axis and the projection surface intersect each other is defined as a projection surface center, and an axis that connects the projection surface center and an edge portion of the projection surface to each other is defined as a second axis, and a tangential line of the edge portion of the projection surface composed of a part of a spherical shape of the video displaying means is defined as a third axis, then an angle made by the first axis and the third axis is an angle at which it is possible to observe a whole of the video from the first viewpoint position, and an angle made by the first axis and the second axis is an axis at which it is possible to observe the projection surface center from the second viewpoint position. Alternatively, in order to solve the above-mentioned problem, in the video displaying means in the present invention, in a case where an axis that passes through a center of an opening surface serving as the concave surface and is perpendicular to the opening surface is defined as a first axis, a point where the first axis and the projection surface intersect each other is defined as a projection surface center, and an axis that connects the projection surface center and an edge portion of the projection surface to each other is defined as a second axis, and a tangential line of the edge portion of the projection surface composed of a part of a spherical shape of the video displaying means is defined as a third axis, then an angle made by the first axis and the third axis is an angle at which it is possible to observe a whole of the video from both of the first viewpoint position and the second viewpoint position.

BRIEF DESCRIPTION OF THE DRAWINGS

FIG. 1 is a perspective view of a display apparatus for an endoscopic operation in an endoscope system to which the present invention is applied.

FIG. 2 is a schematic view of a using example in the endoscopic operation by the endoscope system to which the present invention is applied.

FIG. 3 is a block diagram showing functional configurations of the endoscope system to which the present invention is applied.

FIG. 4 is a view explaining a positional relationship among a viewpoint position of an operator P, a reflecting mirror and a dome type screen in the endoscope system to which the present invention is applied.

FIG. 5 is a perspective view showing another configuration of the endoscope system to which the present invention is applied.

FIG. 6 is a perspective view showing still another configuration of the endoscope system to which the present invention is applied.

FIG. 7 is a perspective view showing still another configuration of the endoscope system to which the present invention is applied.

FIG. 8 is a perspective view showing still another exterior appearance configuration of the endoscope system to which the present invention is applied.

FIG. 9 is a perspective view showing still another exterior appearance configuration of the endoscope system to which the present invention is applied.

FIG. 10 is a view explaining a configuration of an endoscope device in the endoscope system to which the present invention is applied.

FIG. 11 is a view explaining an arrangement relationship at a site of the endoscopic operation into which the endoscope system to which the present invention is applied is introduced.

FIG. 12 is a view showing a relationship between arrangement of a bed, the operator and assistants and a center point on a projection surface at the site of the endoscopic operation.

FIG. 13 is a view showing another relationship between the arrangement of the bed, the operator and the assistants and the center point on the projection surface at the side of the endoscopic operation.

FIG. 14 is a view explaining a shape of the projection surface, which allows the center point of the projection surface to be certainly seen from the operator and the assistants, in the endoscope system to which the present invention is applied.

FIG. 15 is a view explaining a condition where the center point of the projection surface is seen from the operator and the assistants in the endoscope system to which the present invention is applied.

FIG. 16 is a view explaining a shape of the projection surface, which allows a whole of the projection surface to be certainly seen from the operator and the assistants, in the endoscope system to which the present invention is applied.

FIG. 17 is a view explaining a condition where the whole of the projection surface is seen from the operator and the assistants in the endoscope system to which the present invention is applied.

FIG. 18 is a perspective view showing a simulation sample for use in a first operation task.

FIG. 19 is a graph showing, as training results of the first operation task, each forceps reciprocation time when training is performed while showing a two-dimensional video (2D) and each forceps reciprocation time when training is performed while showing a three-dimensional video (3D) on the dome type screen, between which a comparison is made.

FIG. 20 is a box plot showing, as training results of the first operation task, a standard deviation of the respective forceps reciprocation times when the training is performed while showing the two-dimensional videos (2D) and a standard deviation of the respective forceps reciprocation times when the training is performed while showing the three-dimensional videos (3D) on the dome type screen, between which a comparison is made.

FIG. 21 is a box plot showing, as training results of the first operation task, each number of grip failing times when the training is performed while showing the two-dimensional video (2D) and each number of grip failing times when the training is performed while showing the three-dimensional video (3D) on the dome type screen, between which a comparison is made.

FIG. 22 is a perspective view showing a simulation sample for use in a second operation task.

FIG. 23 is a box plot showing, as training results of the second operation task, the respective forceps reciprocation times when the training is performed while showing the two-dimensional videos (2D) and the respective forceps reciprocation times when the training is performed while showing the three-dimensional videos (3D) on the dome type screen, between which a comparison is made.

FIG. 24 is a graph showing, as training results of the second operation task, each number of grip failing times when the training is performed while showing the two-dimensional video (2D) and each number of grip failing times when the training is performed while showing the three-dimensional video (3D) on the dome type screen, between which a comparison is made.

FIG. 25 is a perspective view showing a simulation sample for use in a third operation task.

FIG. 26 is a box plot showing, as training results of the third operation task, shift amounts when the training is performed while showing the two-dimensional videos (2D) and shift amounts when the training is performed while showing the three-dimensional videos (3D) on the dome type screen, between which a comparison is made.

FIG. 27 is a box plot showing, as training results of the third operation task, a standard deviation of the shift amounts when the training is performed while showing the two-dimensional videos (2D) and a standard deviation of the shift amounts when the training is performed while showing the three-dimensional videos (3D) on the dome type screen, between which a comparison is made.

FIG. 28 is a top view showing a simulation sample for use in a fourth operation task.

FIG. 29 is a graph showing, as training results of the fourth operation task, each suture/ligation time when the training is performed while showing the two-dimensional video (2D) and each suture/ligation time when the training is performed while showing the three-dimensional video (3D) on the dome type screen, between which a comparison is made.

FIG. 30 is a graph showing, as training results of the fourth operation task, each number of grip failing times when the training is performed while showing the two-dimensional video (2D) and each number of grip failing times when the training is performed while showing the three-dimensional video (3D) on the dome type screen, between which a comparison is made.

FIG. 31 is a perspective view showing a simulation sample for use in a first recognition task.

FIG. 32 is a box plot showing, as training results of the first recognition task, correct answer rates in the case of using the two-dimensional videos and correct answer rates in the case of using the three-dimensional video, between which a comparison is made.

FIG. 33 is a box plot showing, as training results of the first recognition task, correct answer rates when the training is performed by using a flat monitor and correct answer rates when the training is performed by using the dome type screen, between which a comparison is made.

FIG. 34 is a perspective view showing a simulation sample for use in a second recognition task.

FIG. 35 is a box plot showing, as training results of the second recognition task, correct answer rates in the case of using the two-dimensional videos and correct answer rates in the case of using the three-dimensional videos on the dome type screen, between which a comparison is made.

FIG. 36 is a box plot showing, as training results of the second recognition task, correct answer rates when the training is performed by using the flat monitor and correct answer rates when the training is performed by using the dome type screen, between which a comparison is made.

FIG. 37 is a perspective view showing a simulation sample for use in a third recognition task.

FIG. 38 is a box plot showing, as training results of the third recognition task, correct answer rates in the case of using the two-dimensional videos and correct answer rates in the case of using the three-dimensional videos on the dome type screen, between which a comparison is made.

FIG. 39 is a box plot showing, as training results of the third recognition task, correct answer rates when the training is performed by using the flat monitor and correct answer rates when the training is performed by using the dome type screen, between which a comparison is made.

BEST MODE FOR CARRYING OUT THE INVENTION

A description is made below of an embodiment of the present invention with reference to the drawings.

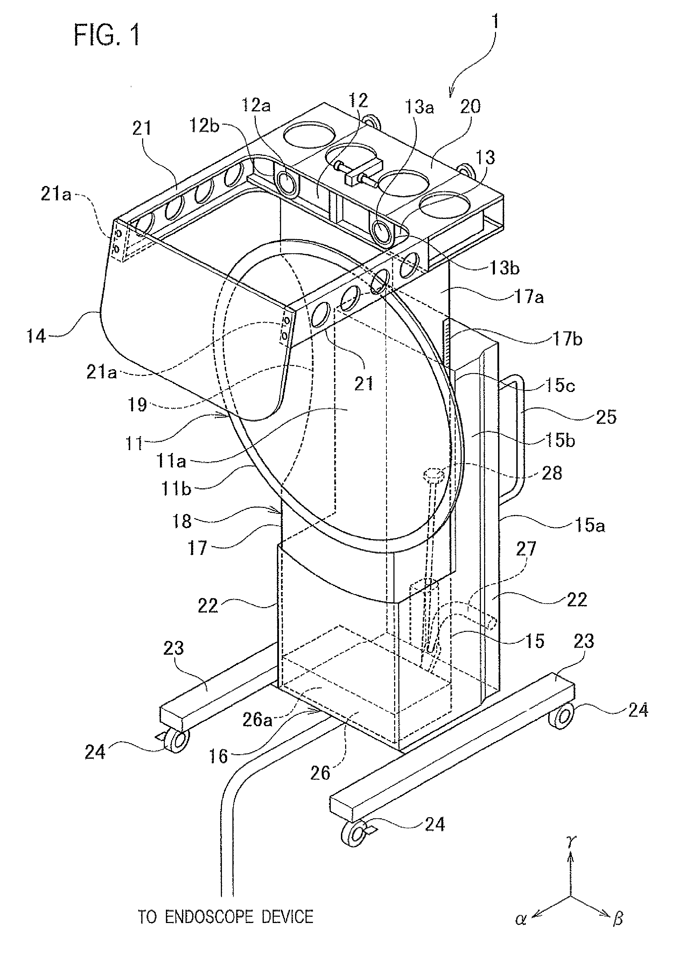

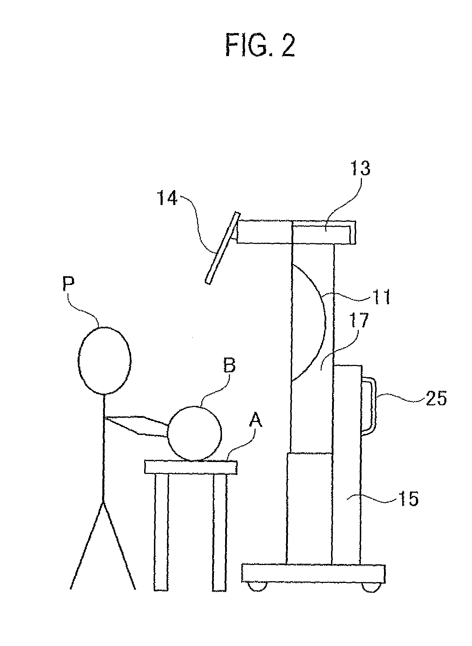

An endoscope system to which the present invention is applied acquires a video of an imaging target in a patient's body cavity in an endoscopic operation, and presents a stereoscopic video of the imaging target in the patient's body cavity to a plurality of persons including an operator (first operator) of the endoscopic operation. For this purpose, this endoscope system has, as an endoscopic operation-use display apparatus 1, a configuration including a dome type screen 11 composed of a part of a sphere, which is as shown in FIG. 1. As shown in Fig. 2, in this endoscope system, a workbench A (such as a bed) is installed in front of the dome type screen 11, and a patient B is laid on the bed A. Moreover, a camera of an endoscope device 2 to be described later is arranged so as to image the patient B, and a video taken by the camera is stereoscopically displayed on the dome type screen 11. The plurality of persons including the operator P perform the endoscopic operation for the patient B while seeing the video displayed on the dome type screen 11.

First, a detailed description is made of the endoscopic operation-use display apparatus 1 in the endoscope system as described above with reference to FIG. 1 to FIG. 7.

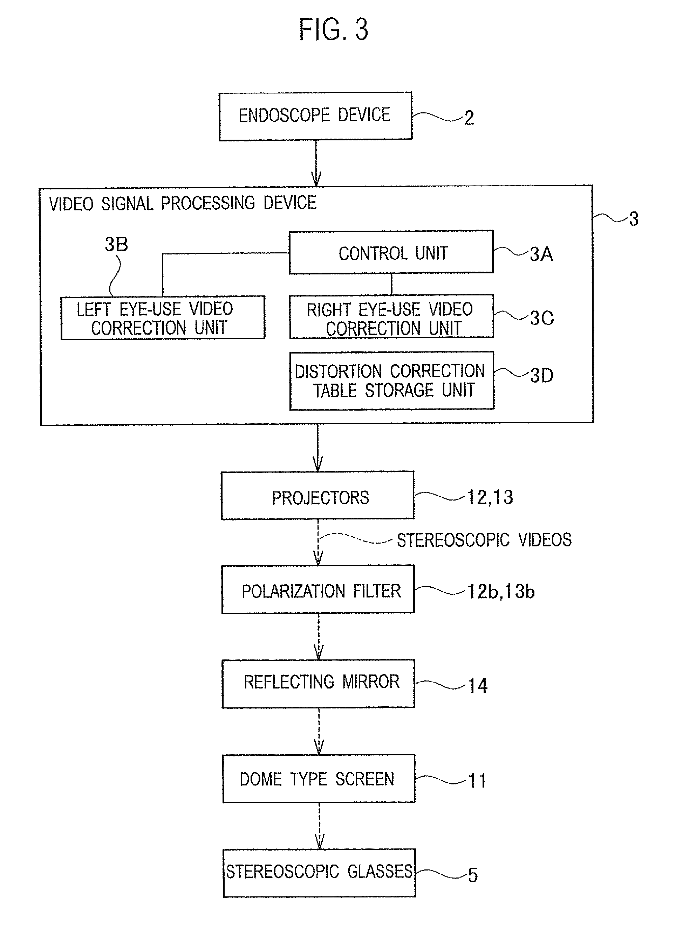

As shown in FIG. 3, this endoscopic operation-use display apparatus 1 is connected to the endoscope device 2 through a video signal processing device 3, and stereoscopically displays the video, which is taken by the endoscope device 2, on the dome type screen 11 without distortion. The plurality of persons including the operator P can perform the endoscopic operation and training for the endoscopic operation while confirming the video, which is taken by the endoscope device 2, by the dome type screen 11 from positions different from one another.

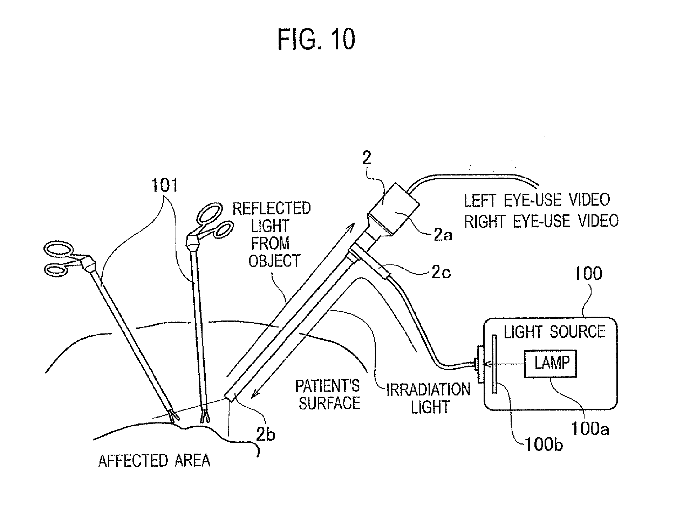

In order to display the stereoscopic video on the dome type screen 11 by the endoscopic operation-use display apparatus 1, the endoscope device 2 includes a camera unit, which takes the video, in a main body portion 2a or tip end portion 2b thereof as shown in FIG. 10. Then, this endoscope device 2 supplies a video signal to the endoscopic operation-use display apparatus 1 through the video signal processing device 3. Note that a configuration of this endoscope device 2 is described later.

This endoscopic operation-use display apparatus 1 includes: projectors 12 and 13 (video projecting means) which emit videos upon receiving video signals; a reflecting mirror 14 that reflects the videos emitted from the projectors 12 and 13; the dome type screen 11 (video displaying means) having a dome type projection surface 11a onto which the videos reflected by the reflecting mirror 14 are projected; a base portion 15 that supports the projectors 12 and 13, the reflecting mirror 14 and the dome type screen 11; and a lifting device 16 that moves the projectors 12 and 13, the reflecting mirror 14 and the dome type screen 11, which are integrated with one another, vertically with respect to the base portion 15.

As shown in FIG. 3, the video signal processing device 3 includes: a control unit 3A that is connected to the endoscope device 2 and performs signal output control for the endoscopic operation-use display apparatus 1, and the like; a left eye-use video correction unit 3B and a right eye-use video correction unit 3C, which perform distortion correction processing for the video signals, which are to be inputted to the projectors 12 and 13, so that the videos can be displayed on the projection surface 11a without distortion when seen from a first viewpoint position of the operator P; and a distortion correction table storage unit 3D that stores therein a distortion correction table for use in the distortion correction processing. These left eye-use video correction unit 3B and right eye-use video correction unit 3C supply a right eye-use video signal and a left eye-use video signal to the projectors 12 and 13.

In order that a left eye-use video can be displayed on the dome type screen 11 without being distorted when seen from the first viewpoint position of the operator, the left eye-use video correction unit 3B refers to the distortion correction table for the left eye-use video signal, and performs distortion correction therefor based on the distortion correction table. Specifically, when two two-dimensional videos (non-stereoscopic videos) corresponding to right and left eyes are supplied to the video signal processing device 3 from the endoscope device 2, and the videos are projected onto the dome type screen 11 from the projector 13, the video signal processing device 3 performs coordinate conversion for the two-dimensional videos so that the videos cannot look distorted on the dome type screen 11 from the first viewpoint position of the operator P.

Note that, for this purpose, the distortion correction table that performs the coordinate conversion for the two-dimensional videos so that the videos cannot look distorted on the dome type screen 11 from the above-described first viewpoint position is created in advance by using correction parameters such as a relative positional relationship among the left eye-use projector 12, the reflecting mirror 14, the viewpoint position (first viewpoint position) of the operator and the dome type screen 11, a shape of the dome type screen 11, and projector characteristics including a specified projection angle and image angle of the left eye-use projector 12. The distortion correction table thus created is stored in advance in the distortion correction table storage unit 3D of the video signal processing device 3. Then, the distortion correction is performed in accordance with the distortion correction table. Moreover, in a similar way to the right eye-use video correction unit 3B, the right eye-use video correction unit 3C also performs the distortion correction in accordance with a distortion correction table created by using correction parameters such as a relative positional relationship among the right eye-use projector 13, the reflecting mirror 14, the viewpoint position of the operator and the dome type screen 11, the shape of the dome type screen 11, and projector characteristics including a specified projection angle and image angle of the right eye-use projector 13. In such a way, the video signal processing device 3 can project, from the projectors 12 and 13, the stereoscopic video having a predetermined parallax between the right eye-use video and the left eye-use video. Note that, in the case of not using the reflecting mirror 14, the distortion correction table is created by omitting a relative position of the reflecting mirror 14 and using correction parameters including a relative positional relationship among the left eye-use projector 12, the viewpoint position of the operator and the dome type screen 11.

Moreover, it is desirable that this endoscope system further include, though not shown, a living body information acquisition unit that acquires patient's living body information such as blood pressure necessary in the endoscopic operation and medical images (such as CT images and MRI images) acquired before and during the operation. Then, this endoscope system projects video light in which the living body information acquired by the living body information acquisition unit is superposed on a stereoscopic video of a patient's affected area. In such a way, the endoscope system allows the operator and the like to visually recognize a variety of information without changing attitudes thereof while seeing the video of the affected area, which is acquired by the endoscope device 2.

Furthermore, at the time when the two two-dimensional video signals corresponding to the left and right eyes and including the videos taken by the endoscope device 2 are inputted to the left eye-use video correction unit 3B and the right eye-use video correction unit 3C, this endoscope system may switch between the following two cases. In one of the cases, the two-dimensional video signal corresponding to the left eye is inputted to the left eye-use video correction unit 3B, and the two-dimensional video signal corresponding to the right eye is inputted to the right eye-use video correction unit 3C. In the other case, the video in either one of the two two-dimensional video signals is inputted to both of the left eye-use video correction unit 3B and the right eye-use video correction unit 3C. By such switching, the video signals corrected by the left eye-use video correction unit 3B and the right eye-use video correction unit 3C may be switched between the stereoscopic video signals and the non-stereoscopic video signals. In such a way, the videos projected from the left eye-use projector 12 and the right eye-use projector 13 can be switched between the non-stereoscopic video and the stereoscopic video. As a trigger of this switching, an arbitrary person may control an action of the video signal processing device 3, for example, in accordance with an instruction of the operator P. In such a way, a selection can be made between the following options. In one of the options, the stereoscopic video is displayed in the case where the endoscopic operation is performed while stereoscopically seeing the affected area, and in the other option, the non-stereoscopic video is displayed in the case where it is not necessary to perform the endoscopic operation while stereoscopically seeing the affected area intendedly. Moreover, a video that facilitates the operator P to perform the endoscopic operation can also be selected.

Still further, a configuration may be adopted, in which a two-dimensional video-use flat screen (not shown) is provided separately in addition to the dome type screen 11, and the dome type screen 11 and the two-dimensional video-use flat screen are switched, whereby the stereoscopic video and the two-dimensional video are switched.

The left eye-use projector 12 as one in the pair receives the left eye-use video signal corrected by the video signal processing device 3, and emits the left eye-use video from a lens 12a. The right eye-use projector 13 as the other in the pair receives the right eye-use video signal outputted from the right eye-use video correction unit and already subjected to the distortion correction, and emits the right eye-use video light from a lens 13a.

Note that this endoscope system adopts a polarization method as a method of allowing the plurality of persons including the operator P to visually recognize the stereoscopic video. Specifically, a left eye-use polarization filter 12b is attached to the lens 12a of the left eye-use projector 12. In a similar way, a right eye-use polarization filter 13b is attached to the lens 13a of the right eye-use projector 13. The left eye-polarization filter 12b and the right eye-use polarization filter 13b transmit therethrough circular polarizations different from each other. The left eye-use video emitted from the left eye-use projector 12 transmits through the left eye-use polarization filter 12b, and the right eye-use video emitted from the right eye-use projector 13 transmits through the right eye-use polarization filter 13b. Note that the respective polarization filters 12b and 13b are not limited to those which transmit the circular polarizations therethrough, and may be those which transmit linear polarizations therethrough. For example, the left eye-use polarization filter 12b may transmit a vertical linear polarization therethrough, and the right eye-use polarization filter 13b may transmit a horizontal linear polarization therethrough.

Meanwhile, at the time of seeing the dome type screen 11, the plurality of persons including the operator P and the assistants (second operators) wear stereoscopic glasses 5. Each pair of the stereoscopic glasses 5 has a polarization filter with the same polarization method as that of the left eye-use polarization filter 12b in a left eye portion thereof, and has a polarization filter with the same polarization method as that of the right eye-use polarization filter 13b in a right eye portion thereof. By seeing the video, which is displayed on the dome type screen 11, trough the stereoscopic glasses 5, the plurality of persons including the operator P can see, by the stereoscopic video, a situation including the affected area, forceps and the like in the patient's body.

The reflecting mirror 14 is installed above viewing fields when the plurality of persons including the operator P look at the center of the dome type screen 11. The reflecting mirror 14 reflects the left eye-use video light and the right eye-use video light, which are emitted from the left eye-use projector 12 and the right eye-use projector 13, toward the projection surface 11a of the dome type screen 11. The endoscopic operation-use display apparatus 1 includes the reflecting mirror 14, whereby it becomes unnecessary to install the projectors 12 and 13 and the dome type screen 11 in line with one another, and the whole of the apparatus can be miniaturized.

As mentioned above, the projection surface 11a of the dome type screen 11 is of the dome type, and is painted with paint, for example, such as a silver paint having a specular reflection effect. In general, this dome type screen 11 is called a silver screen. Note that a shape of the screen is not limited to the dome type, and for example, the screen may be a composite screen composed of a plane and a quadric surface. Even if the screen is the screen having such a shape, the endoscopic operation-use display apparatus 1 can switch the distortion correction table in the video signal processing unit 3, and can thereby switch the video created by the coordinate conversion, and can display, on the projection surface 11a, the video without distortion when seen from the first viewpoint position of the operator P.

Moreover, it is preferable that arithmetic mean roughness of a concave surface on the dome type screen 11 be set within a range where halation owing to inter-reflection is reduced while maintaining high resolution. The reason for the above is as follows. Specifically, in the case of projecting, onto the dome type screen 11, the videos from the respective projectors 12 and 13, then the video toward an end portion side of the dome type screen 11 is irradiated onto an opposite part of the dome type screen 11 owing to the inter-reflection as reflection equal to or more than secondary reflection, which is reflected on a surface irradiated with direct emission light from the projectors 12 and 13. In such a way, the halation occurs that the whole of the dome type screen 11 looks white like flogging. An occurrence degree of the halation owing to the inter-reflection is changed depending on brightness and contrast ratio of the projectors 12 and 13 and the shape of the dome type screen 11. This is because the halation owing to the inter-reflection is particularly prone to occur in the case where the dome type screen 11 is formed into a hemispherical or semicircular shape.

The dome type screen 11 in FIG. 1 in this embodiment has a collar portion (frame) 11b along an outer circumference thereof, and in an inside of the collar portion 11b, the dome type projection surface 11a is formed.

Here, at a site of the endoscopic operation, the position of the dome type screen 11, the viewpoint position of the operator P and the viewpoint positions of the assistants are restrained, and under a condition where there is such a restraint, it is necessary to present a clear stereoscopic video without distortion to at least the operator P. Hence, in the endoscopic operation-use display apparatus 1 in the endoscope system to which the present invention is applied, the shape of the dome type screen 11 is designed so that at least the operator P of the endoscopic operation can see the whole of the projection surface 11a concerned. Still desirably, the shape of the dome type screen 11 may be designed so that not only the operator P but also the assistants and the like, of which viewpoint positions are different from that of the operator P, can see the whole of the projection surface 11a. The shape of the dome type screen 11 is decided by the viewpoint positions of the persons including the operator P and the assistants, and the like; however, details of the shape will be described later.

In the endoscopic operation-use display apparatus 1 as described above, the left eye-use projector 12, the right eye-use projector 13, the reflecting mirror 14 and the dome type screen 11 are assembled integrally with one another by using an attachment member 17, and compose one mobile body 18.

More specifically, the attachment member is formed of a metal plate into a substantially rectangular parallelepiped shape, includes a concave portion 19 on one surface on a front side (positive $\alpha$-axis direction side in FIG. 1) of the endoscopic operation-use display apparatus 1, and includes a projector housing portion 20 on an upper surface thereof. Moreover, a pair of arms 21 are extended from both sides of the projector housing portion 20 toward the front side of the endoscopic operation-sue display apparatus 1.

The dome type screen 11 is fixed to the attachment member 17 in such a manner that the concave projection surface 11a is arranged on the concave portion 19 of the attachment member 17. When the dome type screen 11 is fixed to the attachment member 17, an opening surface of the dome type screen 11 and a front surface of the attachment member 17 become substantially flush with each other. The concave projection surface 11a is arranged on the concave portion 19, whereby the dome type screen 11 is prevented from protruding from the attachment member 17, and the miniaturization of the apparatus can be achieved.

The projector housing portion 20 for the projectors 12 and 13 has a box shape in which at least a side surface on the front side of endoscopic operation-use display apparatus 1 is opened. The projectors 12 and 13 are fixed to an inside of the projector housing portion 20, and the lenses 12a and 13a of the projectors 12 and 13 face to the outside from the side surface thus opened through the polarization filters 12b and 13b.

The reflecting mirror 14 is fixed to tip ends 21a of the pair of arms 21 at a predetermined angle.

The base portion 15 is formed of the into the substantially rectangular parallelepiped shape, includes, on a front side thereof, a box-shaped housing portion 22 in which an upper surface is opened, and includes a pair of leg portions 23 on a lower end thereof. In each of the leg portions 23, casters 24 are individually attached onto both ends thereof in a longitudinal direction. Moreover, handrails 25 are provided on both sides of a back surface 15a of the base portion 15.

A lower portion of the above-mentioned mobile body 18 is housed in the housing portion 22 so as to be freely movable up and down. The lower portion of the mobile body 18 is housed in the box-shaped housing portion 22, whereby the mobile body 18 can be stably maintained at a constant attitude.

Moreover, in the endoscopic operation-use display apparatus 1, the handrails 25 and the casters 24 are provided on the base portion 15. In such a way, the user grips the handrails 25 and pushes the base portion 15, and can thereby move the endoscopic operation-use display apparatus 1 concerned with ease.

Note that, on a side surface 17a of the attachment member 17 for the mobile body 18, a scale 17b is provided, and on a side surface 15b of the base portion 15, a triangle mark 15c is provided. This endoscopic operation-use display apparatus 1 can allow the user to measure a height of the mobile body 18 (screen) by means of the scale 17b and the triangle mark 15c.

The lifting device 16 has, for example, a hydraulic power generation mechanism, and lifts and lowers the mobile body 18. This lifting device 16 includes a drive portion 26, a raising step 27 and a lowering lever knob 28. The drive portion 26 is arranged on a lower surface 22a of the housing portion 22, and the mobile body 18 is fixed to and mounted on an upper surface 26a of the drive portion 26. The raising step 27 and the lowering lever knob 28 are provided on the back surface 15a side of the housing portion 22. When the user steps on the raising step 27 downward from above, the upper surface 26a of the drive portion 26 rises. When the user further steps on the raising step 27 many times, the upper surface 26a further rises. Meanwhile, when the user rotates the lowering lever knob 28 counterclockwise, the upper surface 26a of the drive portion 26 is lowered. When the user rotates the lowering lever knob 28 clockwise, such upward and downward motions of the upper surface 26a of the drive portion 26 are locked. In other words, this lifting device 16 can lift and lower the upper surface 26a of the drive portion 26 vertically with respect to the base portion 15 in such a manner that the user manipulates the raising step 27 and the lowering lever knob 28. In such a way, the mobile body 18 mounted on the upper surface 26a of the drive portion 26 can move vertically with respect to the base portion 15. Note that this lifting device 16 is not limited to the hydraulic one, and other mechanisms such as a spring may be adopted.

Here, in the endoscopic operation-use display apparatus 1, the projectors 12 and 13, the reflecting mirror 14 and the dome type screen 11 are assembled integrally with one another to compose the single mobile body 18. Therefore, even if the mobile body 18 is moved by the lifting device 16, the mutual positional relationship among the projectors 12 and 13, the reflecting mirror 14 and the dome type screen 11 remains fixed. Hence, regardless of the height of the mobile body 18, the video without distortion whenever seen from the first viewing point position of the operator P can be displayed on the dome type screen 11. Specifically, in the case where the projectors 12 and 13, the reflecting mirror 14 and the dome type screen 11 are not integrated with one another, when the height of the dome type screen 11 is changed, the mutual positional relationship among the projectors 12 and 13, the reflecting mirror 14 and the dome type screen 11 is changed, resulting in apprehensions that the video may not be displayed on the center of the dome type screen 11, and that the distorted video when seen from the first viewpoint position of the operator P may be displayed on the screen concerned. Therefore, every time when the height of the dome type screen 11 is changed, the positional relationship among the projectors 12 and 13, the reflecting mirror 14 and the dome type screen 11 must be adjusted, and enormous efforts are required for such position adjustment.

As opposed to the above, in this endoscopic operation-use display apparatus 1, the projectors 12 and 13, the reflecting mirror 14 and the dome type screen 11 are assembled with one another to compose the single mobile body 18, whereby the mutual positional relationship thereamong is fixed. In such a way, even if the height of the mobile body 18 is changed by the lifting device 16, the video without distortion whenever seen from the first viewpoint position of the operator P can be displayed on the dome type screen 11 without adjusting the positions of the projectors 12 and 13, the reflecting mirror 14 and the dome type screen 11.

Moreover, in this endoscopic operation-use display apparatus 1, the mobile body 18 is capable of being lifted and lowered, and accordingly, the dome type screen 11 can be adjusted to a height, at which it is easy to see the video, by the manipulation of the lifting device 16 by the user. At this time, the positional relationship among the projectors 12 and 13, the reflecting mirror 14 and the dome type screen 11 is fixed, and accordingly, even if the mobile body 18 is moved, the video without distortion whenever seen from the first viewpoint position of the operator P is displayed on the dome type screen 11. Moreover, this endoscopic operation-use display apparatus 1 includes the handrails 25 and the casters 24, and accordingly, can be easily moved, for example, from a room to another room by griping the handrails 25 and pushing the base portion 15. Furthermore, the endoscopic operation-use display apparatus 1 is facilitated to pass through, for example, a door of the room by lowering the height of the mobile body 18.

As described above, in response to a stature of the operator P, and the like, the endoscopic operation-use display apparatus 1 can adjust the height of the dome type screen 11, and moreover, can display the video without distortion whenever seen from the first viewpoint position of the operator P even if the height of the dome type screen 11 is changed.

Next, behavior of the endoscope system including the above-mentioned endoscopic operation-use display apparatus 1 are described while referring to FIG. 3.

First, the endoscope device 2 images the patient B. The video signal supplied from the endoscope device 2 is inputted to the video signal processing device 3. This video signal is subjected to the distortion correction by the left eye-use video correction unit 3B and the right eye-use video correction unit 3C so that the videos can be displayed without being distorted when seen from the first viewpoint position of the operator P at the time of being projected onto the dome type screen 11. Such distortion correction processing is processing for converting coordinates of the respective pixels of the two-dimensional video by referring to the distortion correction table, which is stored in the distortion correction table storage unit 3D, in advance in the left eye-use video correction unit 3B and the right eye-use video correction unit 3C, and for thereby creating new videos (right eye-use video signals, left eye-use video signals). Note that, naturally, this distortion correction table is created based on the relative positional relationship among the left eye-use projector 12, the right eye-use projector 13, the reflecting mirror 14, the viewpoint position of the operator P and the dome type screen 11 and on the shape of the dome type screen 11 in order to show the videos without distortion when seen from the viewpoint position of the operator P, which will be described later.

The actions of the left eye-use video correction unit 3B and the right eye-use video correction unit 3C are synchronized with each other by the control unit 3A. The left eye-use video signal and the right eye-use video signal, which are subjected to the distortion correction, are outputted to the left eye-use projector 12 and the right eye-use projector 13, respectively. The left eye-use projector 12 and the right eye-use projector 13 receive the left eye-use video signal and the right eye-use video signal, respectively, and emit the left eye-use video light and the right eye-use video light, respectively.

The left eye-use video light and the right eye-use video light, which are emitted from the left eye-use projector 12 and the right eye-use projector, transmit through the left eye-use polarization filter 12b and the right eye-use polarization filter 13b, respectively, and are made incident onto the reflecting mirror 14. The reflecting mirror 14 reflects the left eye-use video light and the right eye-use video light, which are emitted from the left eye-use projector 12, and the right eye-use projector 13, and projects the left eye-use video light and the right eye-use video light onto the whole of the surface of the dome type screen 11.

The operators (persons) including the operator P wear the stereoscopic glasses 5 at the time of performing the endoscopic operation while seeing the stereoscopic video projected onto the dome type screen 11. Each pair of the stereoscopic glasses 5 has the polarization filter with the same polarization method as that of the left eye-use polarization filter 12b in the left eye portion thereof, and has the polarization filter with the same polarization method as that of the right eye-use polarization filter 13b in the right eye portion thereof. By seeing the video, which is displayed on the dome type screen 11, trough the stereoscopic glasses 5, the plurality of operators including the operator P can visually recognize the video, which is projected onto the dome type screen 11, stereoscopically.

As described above, the endoscope system can adjust the height of the dome type screen 11 in response to the statures of the operator P and the assistants, and the like, and further, can display the video without distortion whenever seen from the first viewpoint position of the operator P even if the height of the dome type screen 11 is changed.

Incidentally, in this embodiment, the videos outputted from the left eye-use projector 12 and the right eye-use projector 13 are reflected on the dome type screen 11 by using the reflecting mirror 14. The endoscopic operation-use display apparatus 1 includes the reflecting mirror 14, whereby it becomes unnecessary to install the left eye-use projector 12, the right eye-use projector 13 and the dome type screen 11 in line with one another, and the video display apparatus can be miniaturized.

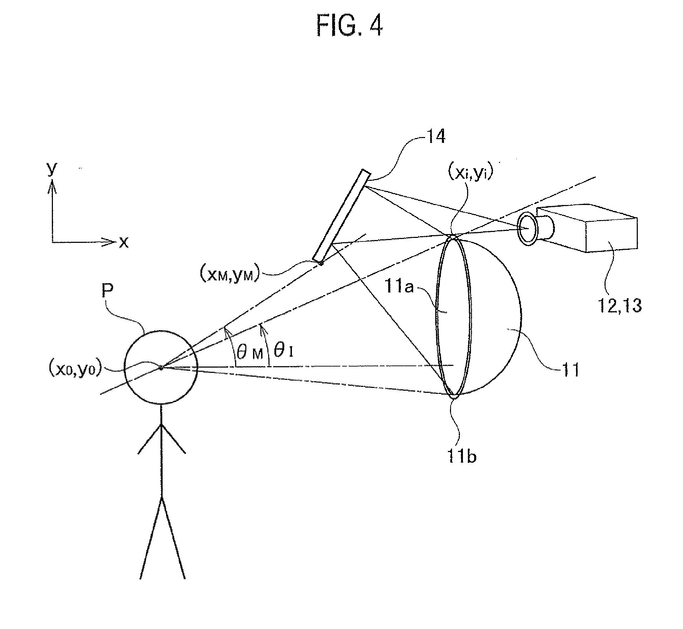

However, in the case where the reflecting mirror 14 is provided, there is an apprehension that the reflecting mirror 14 may block the video since the reflecting mirror 14 comes within sights of such observers. Hence, as shown in FIG. 4, in xy coordinates, in which an x-axis is defined in a line-of-sight direction when the operator P sees the dome type screen 11 horizontally, and a y-axis is defined in a direction perpendicular to the x-axis, it is preferable that the reflecting mirror 14 be installed so that a lower end position $(x_M, y_M)$ of the reflecting mirror 14 concerned satisfy the following Expression.

$$(y_i - y_o) x_M - (x_i - x_o) y_M \leq x_o y_i - x_i y_o \quad \text{(Expression)}$$

Here, $x_o$ and $y_o$ are an x and y coordinate of the preset viewpoint position of the operator P, the assistant or the like, and $x_i$ and $y_i$ are an x and y coordinate of a position of an upper end of the projection surface 11a of the dome type screen 11. Note that the coordinate $(x_i, y_i)$ indicates the upper end of the projection surface 11a, and does not indicate an upper end of the collar portion 11b of the dome type screen 11. Specifically, the coordinate $(x_i, y_i)$ indicates the upper end of such an effective projection surface of the dome type screen 11.

The above-described Expression represents a region where an angle $\theta_M$ at which the operator P looks up the lower end of the reflecting mirror 14 from the preset viewpoint position $(x_o, y_o)$ becomes larger than an angle $\theta_I$ at which the operator P looks up the upper end of the projection surface 11a of the dome type screen 11 from the preset viewpoint position $(x_o, y_o)$. Specifically, the above-described Expression indicates a region where the angle $\theta_M$ is equal to or larger than the angle $\theta_I$ ($\theta_M \geq \theta_I$). Hence, in the case where a position $(x_M, y_M)$ of the lower end of the reflecting mirror 14 satisfies the above-described Expression, the operator P can see the whole of the region of the projection surface 11a without receiving obstruction to the line of sight thereof from the reflecting mirror 14.

Note that, though the videos outputted by the left eye-use projector 12 and the right eye-use projector 13 are projected onto the dome type screen 11 through the reflecting mirror 14 in this embodiment, other configurations may be adopted. For example, the endoscopic operation-use display apparatus 1 may be configured so that the left eye-use projector 12 and the right eye-use projector 13 can be arranged on tip end sides of the arms 21 so as to be opposed to the dome type screen 11, and that the video light emitted from each of the left eye-use projector 12 and the right eye-use projector 13 can thereby be directly projected onto the dome type screen 11.

Specifically, the endoscopic operation-use display apparatus 1 may adopt a configuration of including the left eye-use projector 12, the right eye-use projector 13, the dome type screen 11, the video signal processing device 3,

and the base portion 15 that supports the left eye-use projector 12, the right eye-use projector 13 and the dome type screen 11, in which the left eye-use projector 12, the right eye-use projector 13 and the dome-type screen 11 are assembled integrally with one another to compose a single mobile body (not shown), and the mobile body concerned is moved vertically by the lifting device **8**. Configurations of the video signal processing device 3 and the base portion 15 are similar to those of the video display apparatus of FIG. 1.

Even with such a configuration, in the endoscopic operation-use display apparatus 1, the mobile body becomes capable of being lifted and lowered. Hence, by manipulating the lifting device 8, the user can adjust the dome type screen 11 to the position where it is the easiest to see the video. Moreover, in the endoscopic operation-use display apparatus 1, the positional relationship among the left eye-use projector 12, the right eye-use projector 13 and the dome type screen 11 is fixed. Therefore, even if the mobile body is moved, the endoscopic operation-use display apparatus 1 can display, on the dome type screen 11, the video without distortion whenever seen from the first viewpoint position of the operator P. Note that, even in such a configuration, it is desirable that the dome type screen 11 be arranged on the concave portion 19 of the attachment member 17, and the attachment member be housed in the housing portion 22 so as to be freely movable up and down.

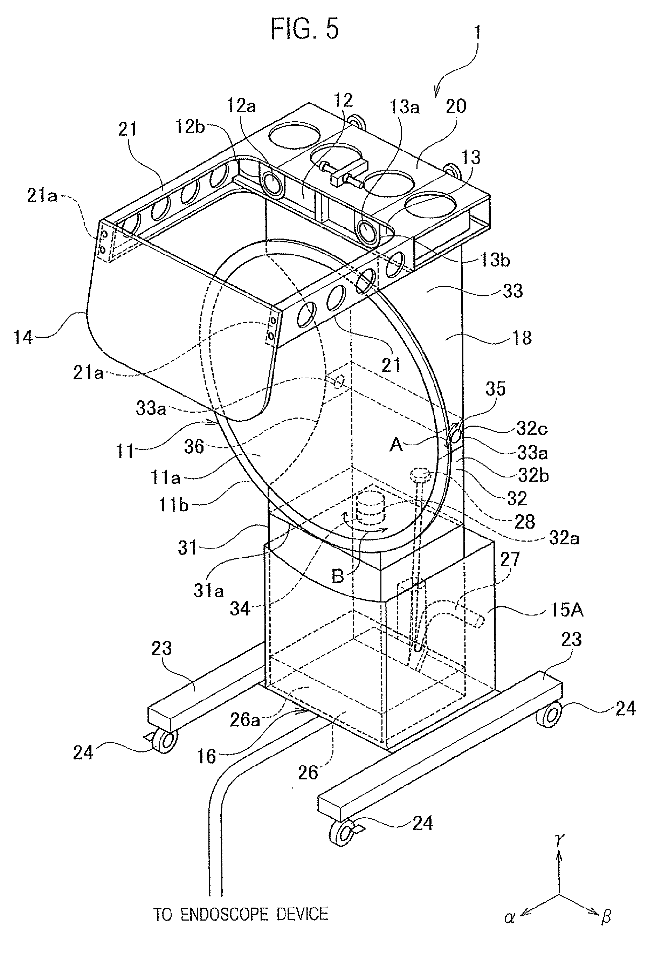

Next, a configuration in which the endoscopic operation-use display apparatus 1 is different in the endoscope system to which the present invention is applied is described with reference to FIG. 5. Except for configurations of the attachment member 17 and the base portion 15, a basic configuration of an endoscopic operation-use display apparatus 1 shown in FIG. 5 is similar to that of the endoscopic operation-use display apparatus 1 shown in FIG. 1. Accordingly, the same reference numerals are assigned to similar portions, and a duplicate description is omitted.

In this endoscopic operation-use display apparatus 1, in addition to be capable of moving vertically, the mobile body 18 is configured to be inclinable with respect to a base portion 15A about an axis perpendicular an up-and-down direction of the endoscopic operation-use display apparatus 1 and parallel to an opening surface of the projection surface 11a (that is, about a β-axis of FIG. 5), and is configured to be rotatable with respect to the base portion 15A about an axis along the up-and-down direction of the endoscopic operation-use display apparatus 1 (that is, about a γ-axis of FIG. 5).

The base portion 15A of this endoscopic operation-use display apparatus 1 is formed into a box shape in which an upper surface is opened, and in a similar way to the endoscopic operation-use display apparatus 1 shown in FIG. 1, the casters 24 are provided on each of the leg portions 23.

An attachment member of the endoscopic operation-use display apparatus 1 is composed of a lower attachment member 31, a center attachment member 32 and an upper attachment member 33. The lower attachment member 31 is formed, for example, of a metal plate into a box shape, and is housed in the base portion 15A so as to be capable of being lifted and lowered. Between the lower attachment member 31 and a bottom surface of the base portion 15A, the drive portion 26 of the lifting device 16 is arranged in a similar way to the endoscopic operation-use display apparatus 1 of FIG. 1. Moreover, a cylindrical shaft portion 34 protrudes from a center of an upper surface 31a of the lower attachment member 31.

The center attachment member 32 is formed, for example, of a metal plate. The center attachment member 32 has a through hole 32a on a lower surface thereof, and is arranged on the lower attachment portion 31 so that the shaft portion 34 of the lower attachment member 31 can penetrate the through hole 32a. In such a way, the center attachment member 32 becomes rotatable with respect to the lower attachment member 31 in a direction of an arrow B of FIG. 5. Moreover, on upper portions of both side surfaces 32b of the center attachment member 32 in a β-axis direction of FIG. 5, through holes 32c are individually formed.

The upper attachment member 33 is formed, for example, of a metal plate. On lower portions of both side surfaces of the upper attachment member 33 in the β-taxis direction of FIG. 5, through holes 33a are individually formed. The upper attachment member 33 is arranged so that lower-side portions of the upper attachment member 33 can overlap upper-side portions of the center attachment portion 32. Positions of the through holes 33a of the upper attachment member 33 coincide with positions of the through holes 32c of the center attachment member 32. In such a positional relationship, bolts 35 are inserted into the through holes 33a and 32c, and the upper attachment member 33 becomes inclinable with respect to the center attachment member 32 about the bolts 35 taken as axes. Specifically, the upper attachment member 33 becomes inclinable in a direction of an arrow A of FIG. 5.

In the endoscopic operation-use display apparatus 1, there is formed a concave portion 36 for arranging the dome type screen 11 in a form of being laid astride a front surface (surface on a positive α-axis direction side in FIG. 5) of the upper attachment member 33 and a front surface of the center attachment member 32. Although being arranged on the concave portion 36, the dome type screen 11 is not fixed to the center attachment portion 32, and is fixed only to the upper attachment portion 33. In order that a back surface of the dome type screen 11 and the concave portion 36 cannot interfere with each other when the upper attachment member 33 is inclined to the center attachment portion 32 side, a predetermined space is provided between the back surface of the dome type screen 11 and the concave portion 36.

In a similar way to the endoscopic operation-use display apparatus 1 of FIG. 1, the projector housing portion 20 is provided on an upper portion of the upper attachment portion 33. In a similar way to the endoscopic operation-use display apparatus 1 of FIG. 1, the left eye-use projector 12, the right eye-use projector 13 and the reflecting mirror 14 are fixed to the upper attachment portion 33. Specifically, in the endoscopic operation-use display apparatus 1 shown in FIG. 5, the left eye-use projector 12, the right eye-use projector 13, the reflecting mirror 14 and the dome type screen 11 are assembled integrally with one another by using the upper attachment member 33 to thereby compose the single mobile body 18.

In the endoscopic operation-use display apparatus 1 configured as described above, when the lifting device 16 lifts and lowers the lower attachment member 31, the mobile body 18 moves vertically with respect to the base portion 15A through the lower attachment member 31 and the center attachment member 32. Hence, by manipulating the lifting device 16, the user can move the mobile body 18 vertically with respect to the base portion 15A. Moreover, the upper attachment member 33 is inclined with respect to the center attachment member 32 manually or by electric power, whereby the mobile body 18 can be inclined with respect to the base portion 15A about the axis perpendicular to the up-and-down direction of the endoscopic operation-use display apparatus 1 and parallel to the opening surface of the projection surface 11a (that is, about the $\beta$-axis of FIG. 5). Furthermore, the center attachment member 32 is rotated with respect to the lower attachment member 31, whereby the mobile body 18 can be rotated with respect to the base portion 15A about the axis along the up-and-down direction of the endoscopic operation-use display apparatus 1 (that is, about the $\gamma$-axis of FIG. 5). The left eye-use projector 12, the right eye-use projector 13, the reflecting mirror 14 and the dome type screen 11 are assembled integrally with one another, and the mutual positional relationship thereamong is fixed. Accordingly, even if the mobile body 18 is moved, inclined and rotated with respect to the base portion 15A, the video without distortion whenever seen from the first viewpoint position of the operator P can be displayed on the dome type screen 11.

In this endoscopic operation-use display apparatus 1, the user can not only move the mobile body 18 vertically, but can also incline and rotate the mobile body 18 with respect to the base portion 15A. In such a way, in accordance with the endoscopic operation-use display apparatus 1, the height and angle of the dome type screen 11 can be adjusted more finely in response to the statures, standing positions and the like of the operator P and the like.

Note that, also in this endoscopic operation-use display apparatus 1, such a configuration may be adopted so that the left eye-use projector 12 and the right eye-use projector 13 can be arranged on the tip end sides of the arms 21 so as to be opposed to the dome type screen 11, and that the videos emitted from the left eye-use projector 12 and the right eye-use projector 13 can thereby be directly projected onto the dome type screen 11.

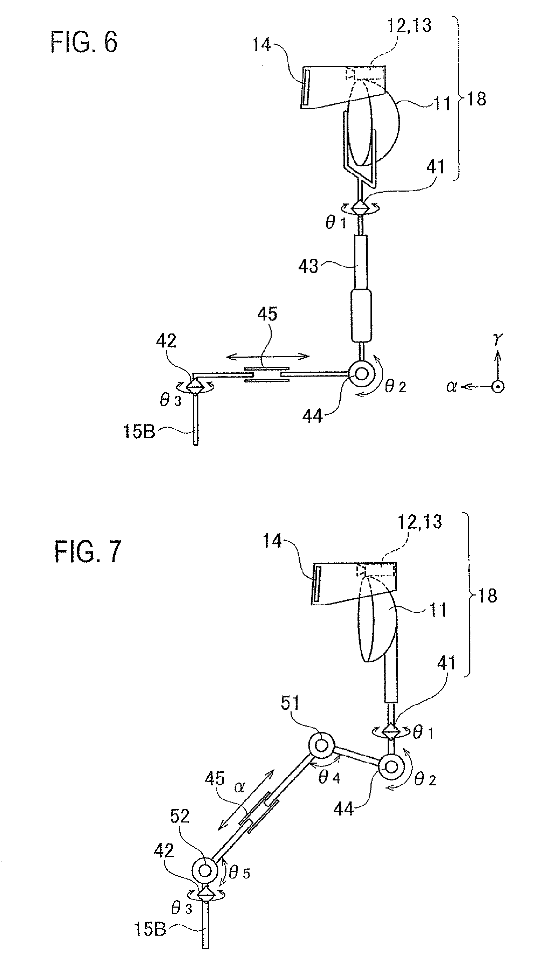

Next, still another configuration of the endoscopic operation-use display apparatus 1 is described with reference to FIG. 6. Note that the same reference numerals are assigned to the same portions as those in the above-mentioned endoscopic operation-use display apparatus 1, whereby a description thereof is omitted.

In a similar way to the endoscopic operation-use display apparatuses 1 shown in FIG. 1 and FIG. 5, in an endoscopic operation-use display apparatus 1 shown in FIG. 6, the left eye-use projector 12, the right eye-use projector 13, the reflecting mirror 14 and the dome type screen 11 are assembled integrally with one another to compose the single mobile body 18.

With regard to a base portion 15B of this endoscopic operation-use display apparatus 1, one end thereof is fixed to the ground (not shown). Between the base portion 15B and the mobile body 18, there are provided: two rotation mechanisms, which are a first rotation mechanism 41 and a second rotation mechanism 42; a lifting mechanism 43; an inclination mechanism 44; an expansion mechanism 45.

The first rotation mechanism 41 is provided between the mobile body 18 and the lifting mechanism 43. This first rotation mechanism 41 can rotate the mobile body 18 with respect to the lifting mechanism 43 in a direction of $\theta_1$ of FIG. 6.

The lifting mechanism 43 is provided between the first rotation mechanism 41 and the inclination mechanism 44. The lifting mechanism 43 can lift and lower the mobile body 18 with respect to the inclination mechanism 44 through the first rotation mechanism 41.

The inclination mechanism 44 is provided between the lifting mechanism 43 and the expansion mechanism 45. This inclination mechanism 44 can inline the mobile body 18 with respect to the expansion mechanism 45 in a direction of $\theta_2$ of FIG. 6 through the lifting mechanism 43 and the first rotation mechanism 41.

The expansion mechanism 45 is provided between the inclination mechanism 44 and the second rotation mechanism 42. This expansion mechanism 45 can move the mobile body 18 with respect to the second rotation mechanism in a direction along an $\alpha$-axis of FIG. 6 through the inclination mechanism 44, the lifting mechanism 43 and the first rotation mechanism 41.

The second rotation mechanism 42 is provided between the expansion mechanism 45 and one end of the base portion 15B. The second rotation mechanism 42 can rotate the mobile body 18 with respect to the one end of the base portion 15B in a direction of $\theta_3$ of FIG. 6 through the expansion mechanism 45, the inclination mechanism 44, the lifting mechanism 43 and the first rotation mechanism 41.

As described above, the endoscopic operation-use display apparatus 1 includes: the two rotation mechanisms 41 and 42; the lifting mechanism 43; the inclination mechanism 44; and the expansion mechanism 45. In such a way, with respect to the base portion 15B, the endoscopic operation-use display apparatus 1 can move the mobile body 18 vertically, can inline the mobile body 18, and can rotate the mobile body 18. No matter how the mobile body 18 may be moved, the left eye-use projector 12, the right eye-use projector 13, the reflecting mirror 14 and the dome type screen 11 are assembled integrally with one another, and the mutual positional relationship thereamong is fixed. Therefore, in accordance with the endoscopic operation-use display apparatus 1, the video without distortion whenever seen from the first viewpoint position of the operator P can be displayed on the dome type screen 11.

In this endoscopic operation-use display apparatus 1, the user can not only move the mobile body 18 vertically in a similar way to the endoscopic operation-use display apparatus 1 shown in FIG. 5, but can also incline and rotate the mobile body 18 with respect to the base portion 15B. In such a way, in accordance with the endoscopic operation-use display apparatus 1, the height and angle of the dome type screen 11 can be adjusted more finely in response to the statures, standing positions and the like of the operator P and assistants of the endoscopic operation.

Note that a combination of the rotation mechanism, the inclination mechanism and the expansion mechanism is not limited to that shown in FIG. 6, and numerous modifications are possible. For example, as shown in FIG. 7, the endoscopic operation-use display apparatus 1 may be configured in such a manner that two rotation mechanisms, which are a third rotation mechanism 51 and a fourth rotation mechanism 52, are further provided to the configuration of FIG. 6, and that the lifting mechanism 43 is removed therefrom.

The third rotation mechanism 51 is provided between the expansion mechanism 45 and the inclination mechanism 44. This third rotation mechanism 51 can rotate the inclination mechanism 44 with respect to the expansion mechanism 45 in a direction of $\theta_4$ of FIG. 7.

The fourth rotation mechanism 52 is provided between the expansion mechanism 45 and the second rotation mechanism 42. This fourth rotation mechanism 52 can rotate the expansion mechanism 45 with respect to the second rotation mechanism 42 in a direction of $\theta_5$ of FIG. 7.

This endoscopic operation-use display apparatus 1 can move the mobile body 18 vertically with respect to the base portion 15B by rotation of the fourth rotation mechanism 52 and by expansion and contraction of the expansion mechanism 45. Specifically, lifting means that can move the mobile body 18 vertically with respect to the base portion 15B is composed of the fourth rotation mechanism 52 and the expansion mechanism 45.

Moreover, in a similar way to the endoscopic operation-use display apparatus 1 shown in FIG. 6, the mobile body 18 can be inclined and rotated with respect to the base portion 15B.

Note that, also in this endoscopic operation-use display apparatus 1, such a configuration may be adopted so that the left eye-use projector 12 and the right eye-use projector 13 can be arranged so as to be opposed to the dome type screen 11, and that the video light emitted from each of the left eye-use projector 12 and the right eye-use projector 13 can thereby be directly projected onto the dome type screen 11.

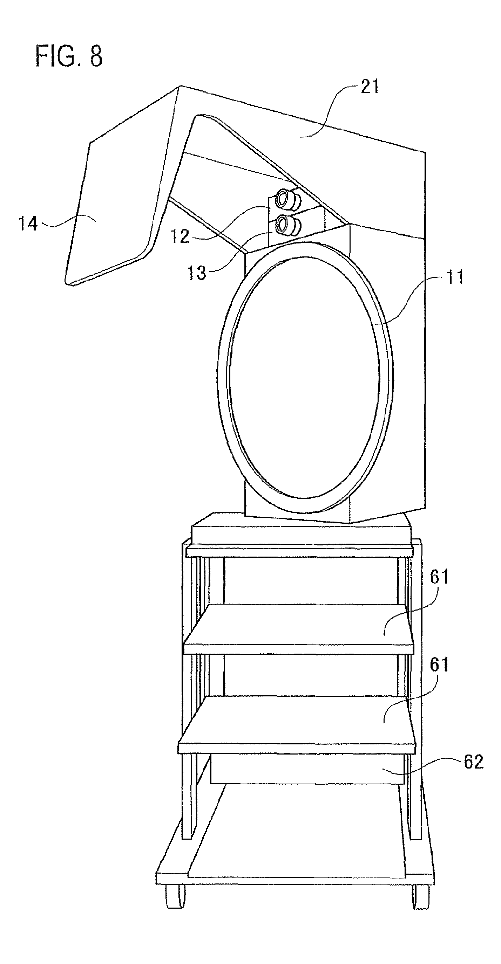

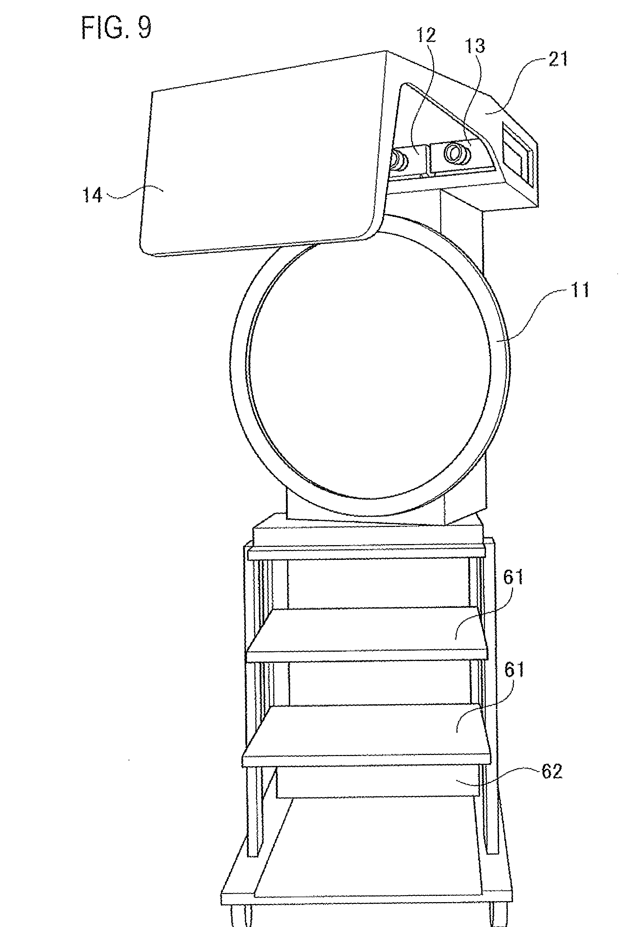

The endoscope system as described above may include, as other forms, endoscopic operation-use display apparatuses 1 having configurations as shown in FIG. 8 and FIG. 9. In both of the endoscopic operation-use display apparatuses 1 shown in FIG. 8 and FIG. 9, work shelves 61 are provided under the dome type screens 11, and below the work shelves concerned, a variety of instrument boxes 62 including the video signal processing devices 3 connected to the endoscope devices 2, and the like are installed. For example, the work shelves are adapted to put thereon varieties of instruments and tools, which are necessary for the endoscopic operation. Moreover, the endoscopic operation-use display apparatus 1 shown in FIG. 8 has a form in which the projectors 12 and 13 are stacked vertically, and the endoscopic operation-use display apparatus 1 shown in FIG. 9 has a form in which the projectors 12 and 13 are stacked horizontally. Note that a dimension of the arms 21, a dimension of the reflecting mirror 14, and the like are optimized based on whether the projectors 12 and 13 are stacked vertically or horizontally.

Next, it is described that, in the endoscopic operation-use display apparatus 1 configured as mentioned above, the dome type screen 11 is configured so that at least the operator P can see the whole of the video on the projection surface 11a and at least the assistants A and B can look at the center of the projection surface 11a at the site of the endoscopic operation.

First, a description is made of positional restraint of the respective persons such as the operator P, the assistants and a cameraman with respect to the position of the dome type screen 11 in the endoscopic operation. In the endoscopic operation in which the above-mentioned endoscope system is used, as shown in FIG. 10, forceps 101 and the tip end portion 2b of the endoscope device 2 are inserted from a body surface of the patient B. In this state, irradiation light emitted from a light source 100 is supplied to the tip end portion 2b of the endoscope device 2.

The light source 100 includes a lamp 100a and a filter 100b. This filter 100b transmits therethrough only a light component with a predetermined wavelength in lamp light emitted by the lamp 100a. This filter 100b is designed so as to transmit therethrough only such a light component that makes a tissue state of the affected area identifiable by emission of irradiation light onto a video imaging range defined by the endoscope device 2. The irradiation light emitted from this light source 100 is guided to the tip end portion 2b through a light introduction portion 2c of the endoscope device 2.

The tip end portion 2b of the endoscope device 2 allows incidence of reflected light in which the emitted irradiation light is reflected on the affected area. In this tip end portion 2b, there are provided: an emission-use lens that emits the irradiation light; and an incidence-use lens that allows the incidence of the reflected light. The reflected light made incident by the incidence-use lens is converted into a video signal by a photoelectric conversion element built in the main body portion 2a or tip end portion 2b of the endoscope device 2, and is supplied to the video signal processing device 3.

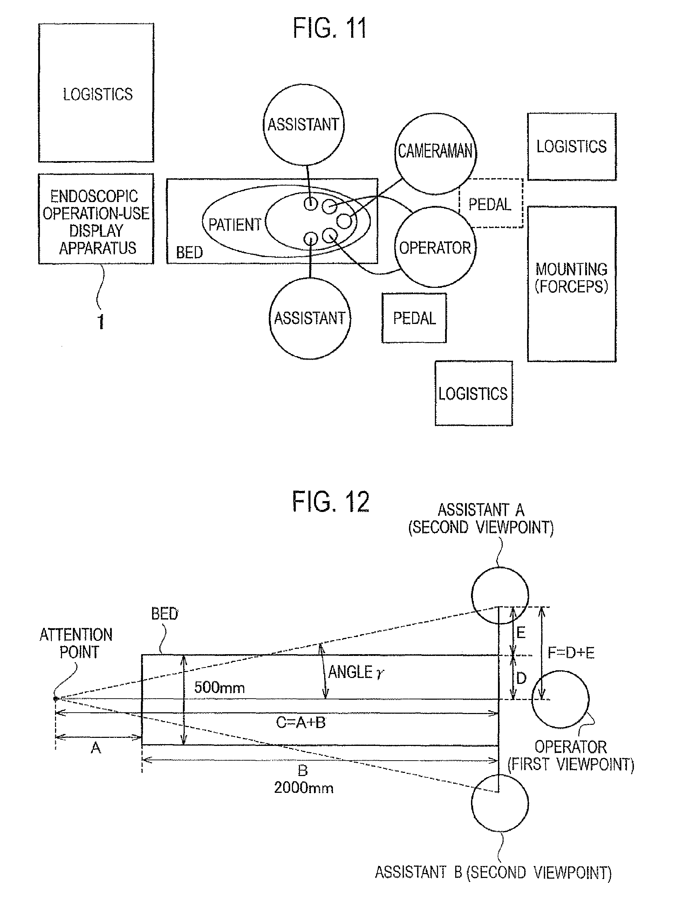

In the endoscopic operation using the endoscope device 2 as described above, as shown in FIG. 11, the dome type screen 11 of the endoscopic operation-use display apparatus 1 and the bed are arranged, and the standing position of the operator P is decided so as to be rightly opposed to the dome type screen 11 concerned. Moreover, in the endoscopic operation, the first assistant and the second assistant, whose standing positions are located on both sides of the bed, are placed. Moreover, in order to image the affected area to be subjected to the operation by the forceps 101 manipulated by the operator P, the cameraman who moves the tip end portion 2b of the endoscope device 2 is placed near the patient.

Under such an environment, it is necessary that the stereoscopic video of the affected area projected onto the dome type screen 11 be clearly seen from the plurality of viewpoint positions of the operator P, the assistants and the cameraman. In particular, it is necessary that the center of the dome type screen 11 be always seen from the operator P.

For this purpose, at the site of the endoscopic operation, as shown in FIG. 12, an angle $\gamma$ becomes necessary in order that all of the members who are the operator P, the assistant A and the assistant B can see an attention point on the dome type screen 11. Specifically, the viewpoint positions of the operator P and the assistants A and B are present within a range where the angle $\gamma$ is equal to 11.31° with respect to the attention point in the case where a distance A from the attention point to a dome type screen 11-side end portion of the bed is 500 mm, a distance B (bed length) from the dome type screen 11-side end portion of the bed to the viewpoints of the assistants A and B in the same direction as a longitudinal side of the bed is 2000 mm, a distance C as the sum of the distance A and the distance B is 2500 mm, a distance D as a half length of a width of the bed is 250 mm, a distance E from an end portion of the bed in the width direction to the assistants A and B is 250 mm, and a distance F as the sum of the distance D and the distance E is 500 mm. In other words, in the case where the center point of the projection surface 11a is defined as the attention point, it is necessary that the dome type screen 11 become a concave surface having an opening portion with the angle $\gamma$ in order to see the video, which is displayed on the center point of the projection surface 11a, from the operator P placed at the first viewpoint position and from the assistants A and B placed at the second viewpoint positions.

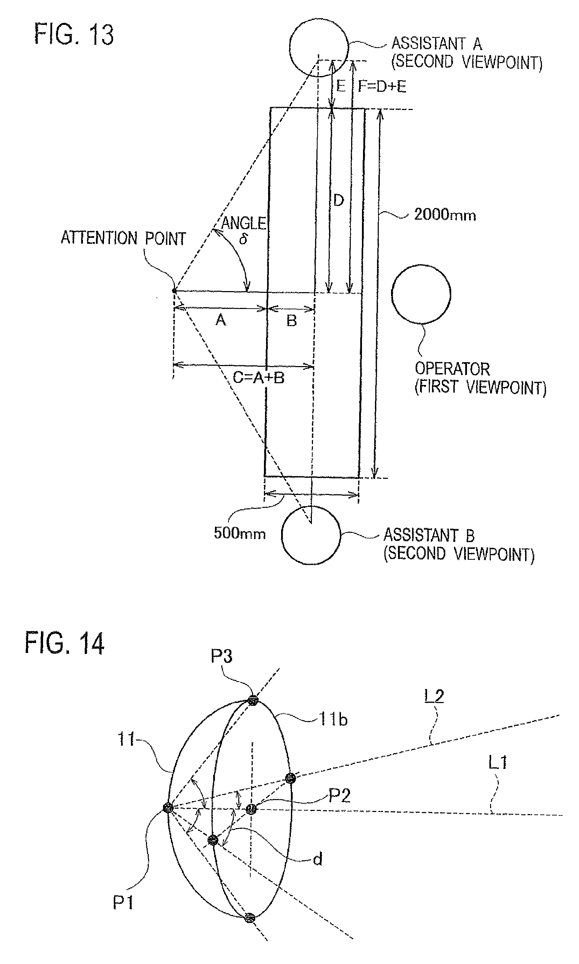

Moreover, at the site of the endoscopic operation, as shown in FIG. 13, there is also a case where the bed is arranged in a horizontally oriented manner with respect to the attention point, the first viewpoint position of the operator P is arranged so as to be substantially rightly opposed to the attention point, and the second viewpoint positions of the assistants A and B are arranged on both ends of the bed in the longitudinal direction. Under such an environment, an angle $\delta$ becomes necessary in order that all of the members who are the operator P, the assistant A and the assistant B can see the attention point on the dome type screen 11. Specifically, the viewpoint positions of the operator P and the assistants A and B are present within a range where the wangle $\delta$ is equal to 59.04° with respect to the attention point in the case where a distance A from the attention point to a dome type screen 11-side end portion of the bed is 500 mm, a distance B (a half length of the width of the bed) from the dome type screen 11-side end portion of the bed to an intersection of a line extended therefrom in the width direction of the bed and a line extended from the viewpoints of the assistants A and B in the longitudinal direction of the bed is 250 mm, a distance C as the sum of the distance A and the distance B is 750 mm, a distance D as a half of the width of the bed is 250 mm, a distance E from the end portion of the bed in the width direction to the assistants A and B is 250 mm, and a distance F as the sum of the distance D and the distance E is 1250 mm. In other words, in the case where the center point of the projection surface 11a is defined as the attention point, it is necessary that the dome type screen 11 become a concave surface having an opening portion with the angle $\delta$ in order to see the video, which is displayed on the center point of the projection surface 11a, from the operator P placed at the first viewpoint position and from the assistants A and B placed at the second viewpoint positions.

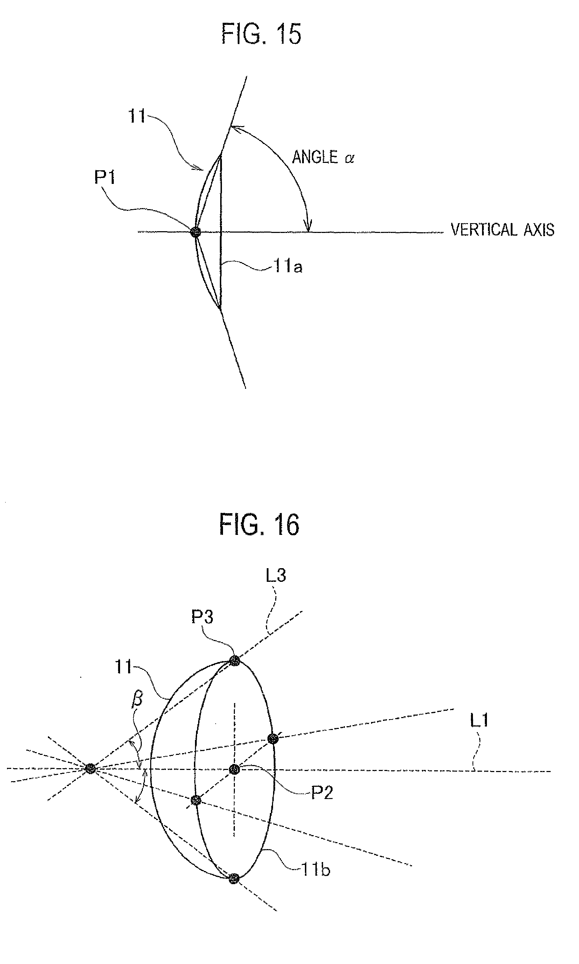

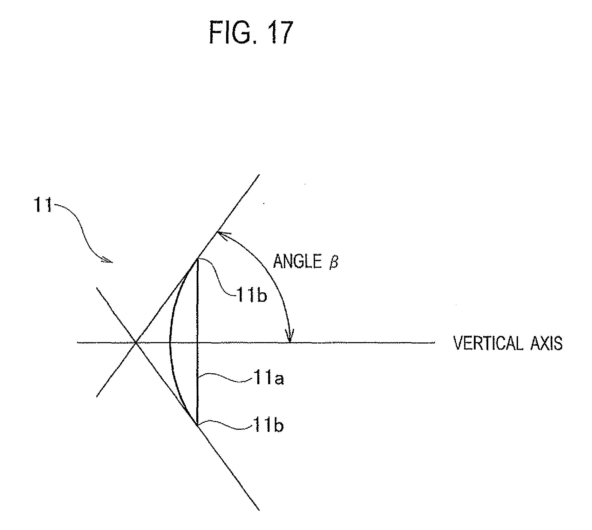

As described above, in order to see the video on the projection surface 11a, a shape of the concave surface of the projection surface 11a is restricted. In this connection, in the endoscope system to which the present invention is applied, the shape of the projection surface 11a that directs the concave surface toward the operator P (first operator) and the assistants A and B (second operators) is made to satisfy an angle $\alpha$ as shown in FIG. 14 and FIG. 15 and an angle $\beta$ as shown in FIG. 16 and FIG. 17.

The angle $\alpha$ is adjusted to such an angle at which persons present in a range of the angle $\alpha$ concerned with respect to the dome type screen 11 can observe the center of the projection surface of the dome type screen 11. Meanwhile, the angle $\beta$ is adjusted to such an angle at which persons present in a range of the angle $\beta$ concerned with respect to the dome type screen 11 can observe the whole of the video of the dome type screen 11 concerned.

Here, in this endoscope system, a range where the viewpoint position of the operator P is present is determined, and a range where the viewpoint positions (second viewpoint positions) of the assistants A and B is determined. Hence, in the endoscope system to which the present invention is applied, the dome type screen 11 has such a shape that allows the viewpoint position of the operator P to remain with the range of the angle $\beta$ and allows the viewpoint positions of the assistants A and B to remain within the angle $\alpha$. Alternatively, in the endoscope system to which the present invention is applied, the dome type screen 11 has such a shape that allows both of the viewpoint positions of the operator P and the assistants A and B to remain within the angle β.

First, the angle α is described.

As shown in FIG. 14, a perpendicular axis L1 that passes through a center point P2 of the opening surface serving as the concave surface concerned and is perpendicular to the opening surface concerned is defined as a first axis. Moreover, a point where the perpendicular axis L1 as the first axis concerned and the projection surface 11a intersect each other is defined as a projection surface center point P1. Furthermore, a center-edge connection axis L2 that connects the projection surface center point P1 and an edge portion P3 of the projection surface 11a to each other is defined as a second axis. In this case, the projection surface 11a. is formed into such a shape that the angle α made by the perpendicular axis L1 (first axis) and the center-edge connection axis L2 (second axis) can be an angle at which the center of the projection surface 11a can be observed from the second viewpoint positions of the assistants A and B.

Specifically, in order to make it possible to use the endoscopic operation-use display apparatus 1 at both of the site of the endoscopic operation as shown in FIG. 12 and the site of the endoscopic operation as shown in FIG. 13, it is necessary that the projection surface 11a be configured so that the angle α made by the perpendicular axis L1 (first axis) and the center-edge connection axis L2 (second axis) can be larger than 59.04 degrees and remain within a range of the maximum angle at which the projection surface 11a is recognizable as the concave surface. The maximum angle at which the projection surface 11a is recognizable as the concave surface excludes a plane in terms of meaning. It is desirable that the maximum angle be such an angle at which the plurality of operators including the operator P and the assistants can obtain a correct depth perception with respect to the stereoscopic vide concerned in the case of displaying the stereoscopic video on the projection surface 11a by projecting the left eye-use video light and the right eye-use video light thereonto.

The projection surface 11a is configured as described above, whereby the assistants A and B can look at the center of the projection surface 11a if the second viewpoint positions of the assistants A and B are arranged within the range of the angle α as shown in FIG. 15. Note that the operator P can also look at the center of the projection surface 11a in a similar way at the time of moving into the range of the angle α.

Specifically, if an opening diameter of the concave surface is set at 600 mm, and a depth from the opening surface of the concave surface concerned to the projection surface center point P1 is set at 46 mm, then the angle α from the perpendicular axis L1 to the center-edge connection axis L2 becomes 81.28 degrees. As another specific example, if the opening diameter of the concave surface is set at 600 mm, and the depth from the opening surface of the concave surface concerned to the projection surface center point P1 is set at 100 mm, then the angle α from the perpendicular axis L1 to the center-edge connection axis L2 becomes 71.57 degrees. As still another specific example, if the opening diameter of the concave surface is set at 600 mm, and the depth from the opening surface of the concave surface concerned to the projection surface center point P1 is set at 200 mm, then the angle α from the perpendicular axis L1 to the center-edge connection axis L2 becomes 56.31 degrees.

Based on the specific examples as described above, the maximum angle at which the projection surface 11a is recognizable as the concave surface, the maximum angle representing a range where it is possible to arrange the first viewpoint position of the operator P and the second viewpoint positions of the assistants A and B, from which the center of the projection surface 11a can be seen, is set at 82 degrees. Meanwhile, the minimum range where it is possible to arrange the first viewpoint position of the operator P and the second viewpoint positions of the assistants A and B, from which the center of the projection surface 11a can be seen, is set at 11 degrees in the example of the site of the endoscopic operation shown in FIG. 12. Based on these matters, it is desirable that the projection surface 11a be configured so that the angle made by the perpendicular axis L1 (first axis) and the center-edge connection axis L2 (second axis) can be set within a range from 11 degrees to 82 degrees.

In accordance with the endoscope system as described above, even if the viewpoint positions of the persons who see the stereoscopic video and the position of the dome type screen 11 are restrained, the shape of the projection surface 11a can be adjusted so that the viewpoint positions of the persons who see the stereoscopic video can be located within the range of the angle α between the perpendicular axis L1 and the center-edge connection axis L2. In such a way, if the viewpoint positions of the persons who see the stereoscopic video are determined to some extent, the endoscope system can allow the persons to certainly look at the center of the projection surface 11a.

Hence, in accordance with this endoscope system, even in the case where the position and the attitude, at which the dome type screen 11 can be arranged, and the arrangement of all the members including the operator P and the assistants A and Bare restricted by the arrangement of the bed, the patient B and other instruments, which are as shown in FIG. 12 and FIG. 13, the angle α between the perpendicular axis L1 and the center-edge connection axis L2 is adjusted, whereby the persons located in the range of the angle α can be allowed to certainly visually recognize the center point of the projection surface 11a. Moreover, the angle α of the projection surface 11a is adjusted so as to cover a range where the viewpoint positions move, whereby the center point of the projection surface 11a can be certainly shown.

Moreover, in accordance with this endoscope system, the distortion correction table for performing the distortion correction processing based on the first viewpoint position of the operator P is stored in advance in the distortion correction table storage unit 3D, and the stereoscopic video is displayed after performing the distortion correction processing therefor. Accordingly, the center position of the video without distortion can be allowed to be certainly visually recognized from the first viewpoint position of the operator P. In such a way, the endoscope system removes such anxieties that the center of the projection surface 11a may become invisible from all the members including the operator P and the assistants A and B, and can reduce a stress in the endoscopic operation.

Next, the angle β is described.

In this endoscope system, as shown in FIG. 16, the projection surface 11a is composed of a part of a spherical surface, a tangential line L3 of the edge portion of the projection surface 11a is defined as a third axis, and an angle made by the perpendicular axis L1 (first axis) and the tangential line L3 (third axis) is defined as an angle β at which the whole of the video can be observed from the first viewpoint position of the operator P. Moreover, in this endoscope system, the angle made by the perpendicular axis L1 (first axis) and the tangential line L3 (third axis) is defined as an angle β at which the whole of the video can be observed from both of the first viewpoint position of the operator P and the second viewpoint positions of the assistants A and B.

The projection surface 11a as described above is configured, whereby all the members who are the operator P and the assistants A and B can see the whole of the video on the projection surface 11a if the first viewpoint position of the operator P and the second viewpoint positions of the assistants A and B are arranged within a range of the angle β as shown in FIG. 17.

Specifically, if the opening diameter of the concave surface is set at 600 mm, and the depth from the opening surface of the concave surface concerned to the projection surface center point P1 is set at 46 mm, then the angle β becomes 72.54 degrees. As another specific example, if the opening diameter of the concave surface is set at 600 mm, and the depth from the opening surface of the concave surface concerned to the projection surface center point P1 is set at 100 mm, then the angle β becomes 53.13 degrees. As still another specific example, if the opening diameter of the concave surface is set at 600 mm, and the depth from the opening surface of the concave surface concerned to the projection surface center point P1 is set at 200 mm, then the angle β becomes 22.62 degrees. Based on these matters, it is desirable that the projection surface 11a be configured so that the angle made by the perpendicular axis L1 (first axis) and the tangential line L3 (third axis) can be set within a range from 11 degrees to 73 degrees.

In accordance with the endoscope system as described above, even if the first viewpoint position of the operator P, the second viewpoint positions of the assistants A and B and the position of the dome type screen 11 are restrained, the shape of the projection surface 11a can be adjusted so that the first viewpoint position of the operator P and the second viewpoint positions of the assistants A and B can be located within the range of the angle β between the perpendicular axis L1 and the tangential line L3. In such a way, if the first viewpoint position of the operator P and the second viewpoint positions of the assistants A and B are determined to some extent, the endoscope system can allow these persons to certainly see the whole of the video on the projection surface 11a.

Hence, in accordance with this endoscope system, even in the case where the position and the attitude, at which the dome type screen 11 can be arranged, and the arrangement of all the members including the operator P and the assistants A and B are restricted by the arrangement of the bed, the patient B and the other instruments, which are as shown in FIG. 12 and FIG. 13, the angle β between the perpendicular axis L1 and the tangential line L3 is adjusted, whereby the operator P and the assistants A and B can be allowed to certainly visually recognize the whole of the video on the projection surface 11a. Moreover, the angle β of the projection surface 11a is adjusted so as to cover the range where the viewpoint positions of the operator P and the assistant A and B move, whereby the whole of the video on the projection surface 11a can be certainly shown to the operator P and the assistants A and B.

Moreover, in accordance with this endoscope system, the distortion correction table for performing the distortion correction processing based on the first viewpoint position of the operator P is stored in advance in the distortion correction table storage unit 3D, and the stereoscopic video is displayed after performing the distortion correction processing therefor. Accordingly, the whole of the video without distortion can be allowed to be certainly visually recognized from the first viewpoint position of the operator P.

Next, a description is made of a difference in effect, in the above-mentioned endoscope system, between the case of using a two-dimensional video (2D) as means for presenting the video by a general 20-inch Trinitron monitor and the case of using a three-dimensional video (3D) as the means for presenting the video by the dome type screen 11 in which the concave surface shape is adjusted as in the endoscopic operation-use display apparatus 1 to which the present invention is applied.

In this description of the effects, an endoscopic operation training system is used, in which, in place of the patient B shown in FIG. 2, a simulation sample that briefly simulates the affected area of the patient as an operation target is mounted on the workbench A. This endoscopic operation training system includes an endoscopic operation-use display apparatus 1 and a video signal processing device 3, which are similar to those of the above-mentioned endoscope system, and makes the operator P wear the stereoscopic glasses 5. Then, operation instruments such as the forceps manipulated by the operator P at the first viewpoint position and the tip end portion 2b of the endoscope device 2 are inserted into the simulation sample, and the manipulation of the forceps is imaged by the endoscope device 2, and is then displayed on the dome type screen 11 of the endoscopic operation-use display apparatus 1.

As training to be performed by the endoscopic operation training system as described above, there are mentioned: first to fourth operation tasks as training for manipulating the forceps; and first to third recognition tasks for accurately recognizing a front and rear positional relationship between targets in the video displayed on the dome type screen 11. A description is made below of the respective tasks and the effects of the endoscope system.

[First Operation Task]

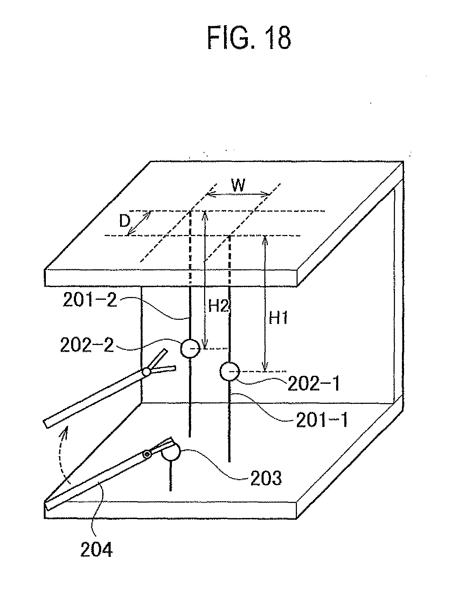

For the first operation task, a simulation sample as shown in FIG. 18 was used. In this simulation sample, sensors 202-1 and 202-2 are provided on a plurality of stringy targets 201-1 and 201-2 in which distances from an imaging position of the endoscope device 2 differ from each other by D. Moreover, a reference position contact sensor 203 is provided between the operator P and the sensors 202-1 and 202-2. The first operation task is training for gripping the sensors 202-1 and 202-2 by forceps 204 and reciprocating the forceps 204 between the reference position contact sensor 203 and the sensors 202-1 and 202-2.

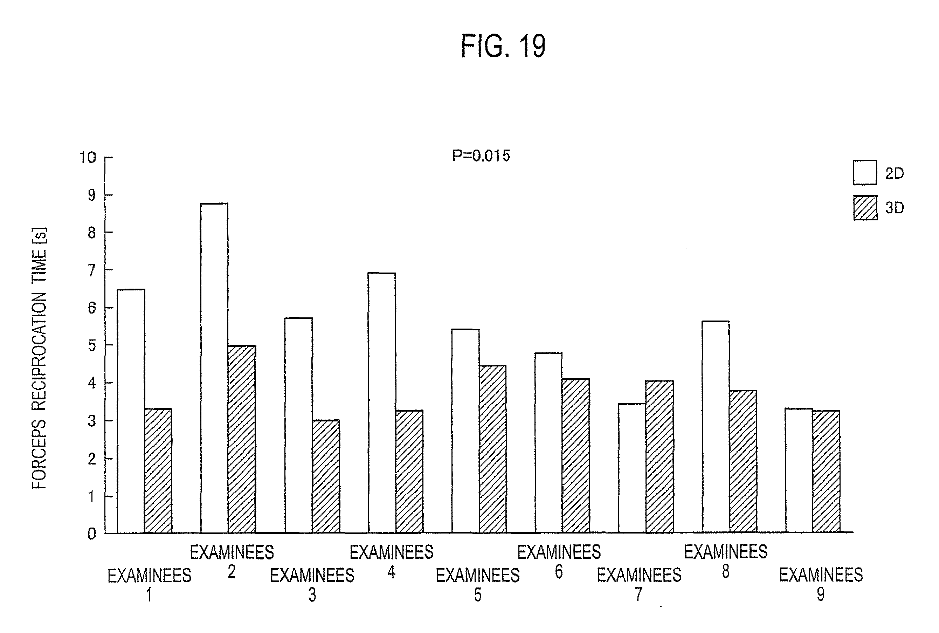

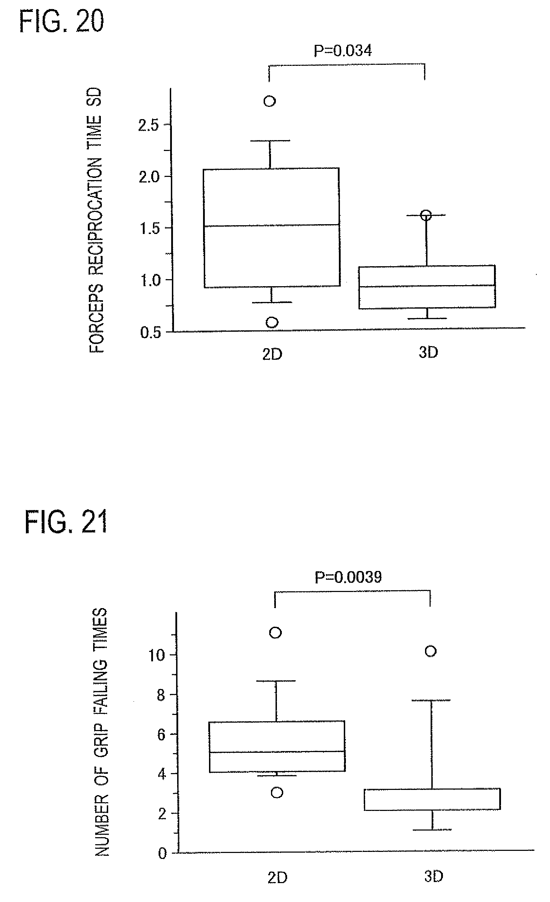

As results of the first operation task, FIG. 19 shows a forceps reciprocation time [sec] for each of examinees (operators P), FIG. 20 shows a standard deviation of the respective forceps reciprocation times [sec], and FIG. 21 shows each number of grip failing times [number of times] for the targets. Note that the training results are acquired in such a manner that a plurality of the operators P perform the first operation task. Here, "P" shown in FIG. 19 is a value obtained by an analysis method called "Wilcoxon Signed Rank Test", and "P" shown in FIG. 20 and FIG. 21 is values obtained by an analysis method called "Mann-Whitney-U Test". The matter that the value of P is 0.05 or less represents that a difference is recognized between the comparison targets. As apparent from FIG. 19, in the case of displaying a situation in the simulation sample by the three-dimensional video (3D), the forceps reciprocating time is shorter than in the case of displaying the situation concerned by the two-dimensional video (2D), and based on the values of P, the difference is recognized between the results of both of the cases.

Moreover, as shown in FIG. 20, in the case of the three-dimensional video (3D), variations of the forceps reciprocating time, which correspond to the operators P, are smaller and the forceps reciprocating time is also shorter than in the case of the two-dimensional video (2D). Moreover, the value of P, which is obtained by the Mann-Whitney-U Test also becomes 0.034. Note that, when a box plot shown in FIG. 21 is described by using the case of the two-dimensional video (2D), values in the vicinities of 2.6 [sec] and 0.5 [sec] are mild outliers, values in the vicinities of 2.25 [sec] and 0.75 [sec] are the minimum and maximum values, values in the vicinities of 0.9 [sec] and 2.0 [sec] are first and third quartiles, and a value in the vicinity of 1.5 [sec] is a median.

Moreover, as shown in FIG. 21, in the case of the three-dimensional video (3D), the number of grip failing times, which corresponds to the operators P, is smaller and the variations are also smaller than in the case of the two-dimensional video (2D). Moreover, the value of P, which is obtained by the Mann-Whitney-U Test also becomes 0.0039.

[Second Operation Task]

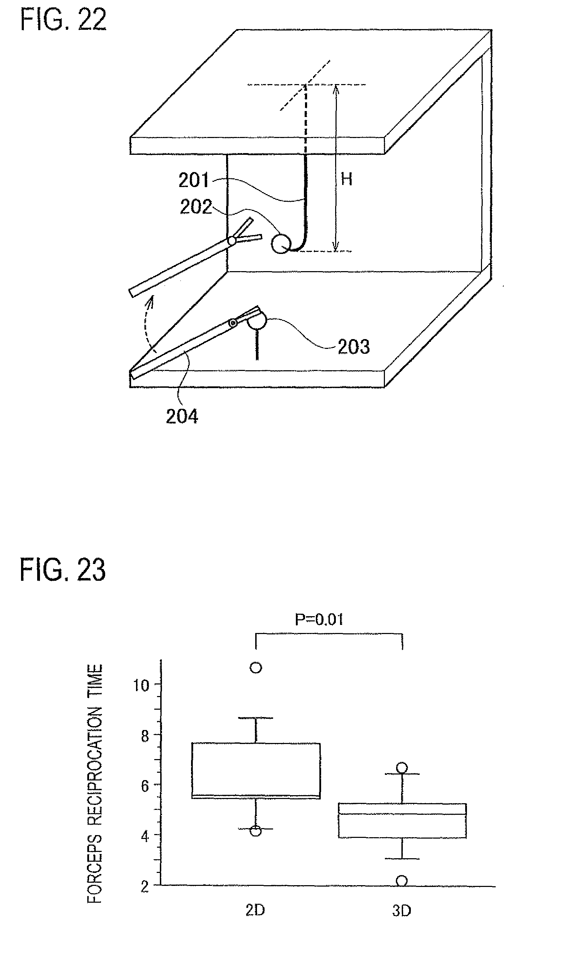

For the second operation task, a simulation sample as shown in FIG. 22 was used. The second operation task is training for allowing the operators P to grip a stringy target 201 by manipulating the forceps 204 and to reciprocate the forceps 204 between a sensor 202 and the reference position contact sensor 203.

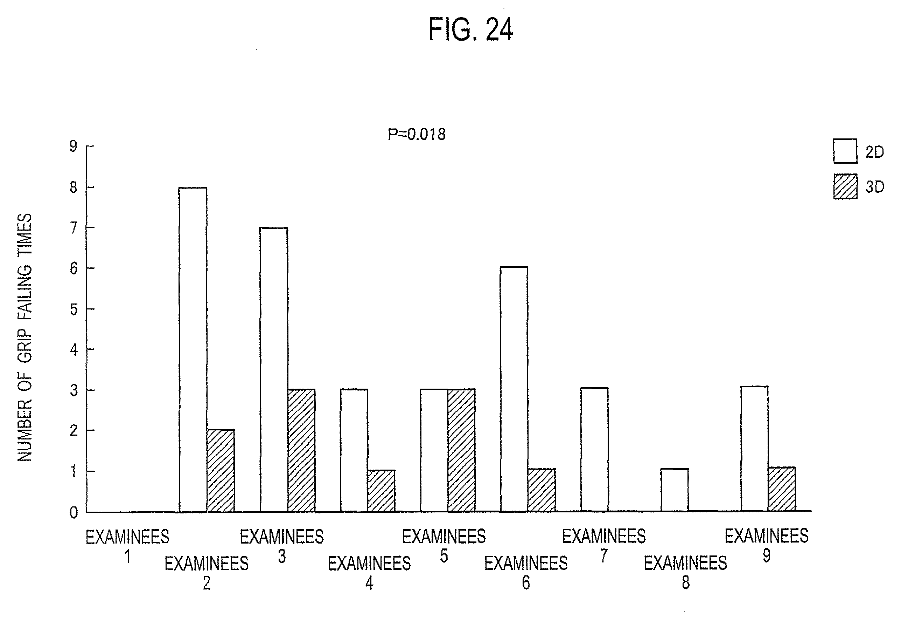

As results of the second operation task, FIG. 23 shows a forceps reciprocation time [sec], and FIG. 24 shows each number of grip failing times [number of times] for the targets. Note that the training results were acquired in such a manner that the plurality of operators P performed the second operation task.

As apparent from FIG. 23, in the case of the display by the three-dimensional video (3D), the forceps reciprocating time is shorter than in the case of the display by the two-dimensional video (2D). Note that the value of P is also 0.01. Moreover, as apparent from FIG. 24, in the case of the display by the three-dimensional video (3D), the number of grip failing times is smaller than in the case of the display by the two-dimensional video (2D). Note that the value of P is also 0.018.

[Third Operation Task]

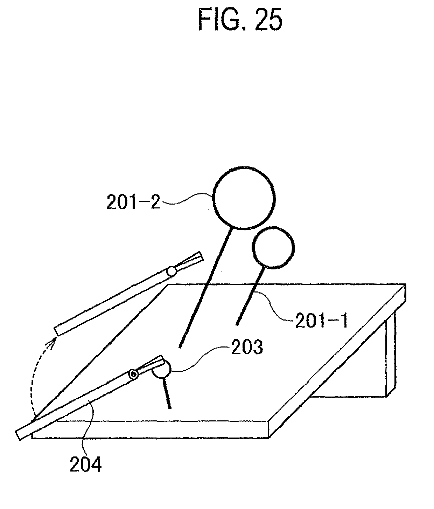

For the third operation task, a simulation sample as shown in FIG. 25 was used. The third operation task is training for allowing the forceps 204 to pass through two annular targets 201-1 and 201-2 different from each other in distance from the operator P, and reciprocating the forceps 204 between the reference position contact sensor 203 and these annular targets 201-1 and 201-2.

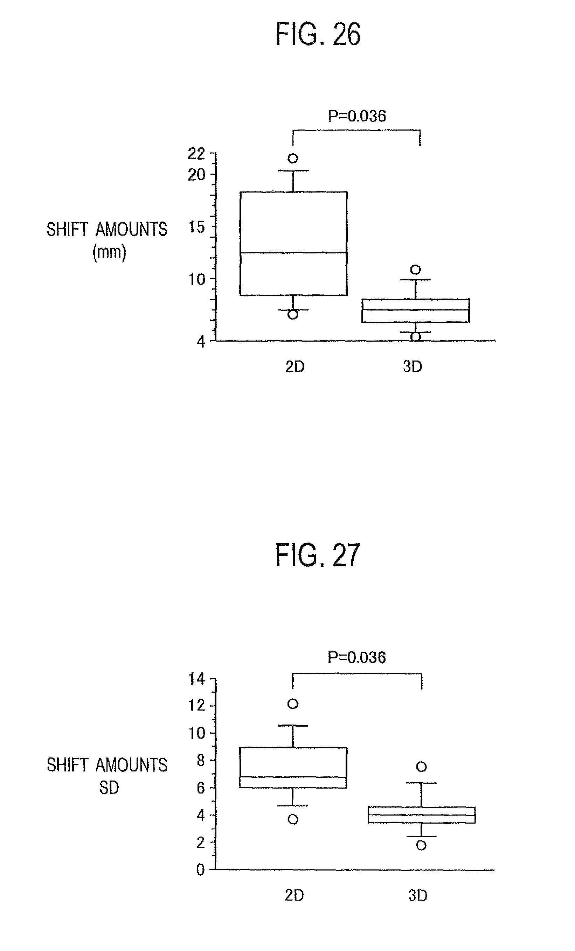

As results of the third operation task, FIG. 26 shows shift amounts of the forceps 204 from centers of annular portions of the targets 201-1 and 201-2, and FIG. 27 shows a standard deviation of the shift amounts. Note that the training results were acquired in such a manner that the plurality of operators P performed the third operation task.

As apparent from FIG. 26, in the case of the display by the three-dimensional video (3D), the shift amounts are smaller and variations thereof are also smaller than in the case of the display by the two-dimensional video (2D). Note that the value of P is also 0.036. Moreover, as apparent from FIG. 27, in the case of the display by the three-dimensional video (3D), the standard deviation of the shift amounts is smaller and variations thereof are also smaller than in the case of the display by the two-dimensional video (2D). Note that the value of P is also 0.036.

[Fourth Operation Task]

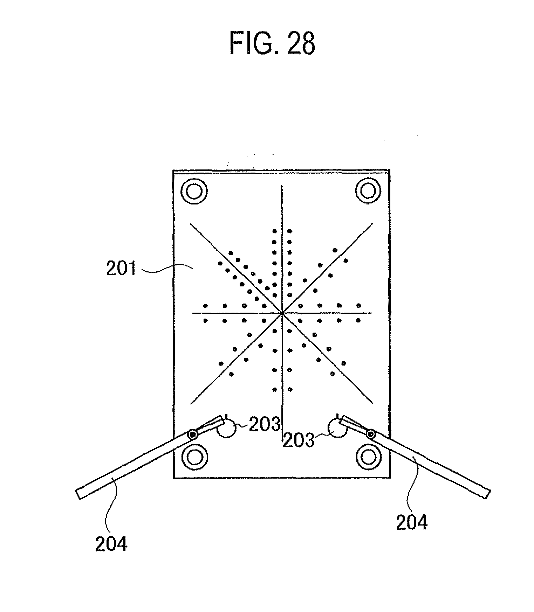

For the fourth operation task, a simulation sample as shown in FIG. 28 was used. The fourth operation task is training for allowing the operators P to perform a suture/ligation operation by manipulating the forceps 204 to a suture practice board on which a plurality of target points are written, and for measuring a time by the reference position contact sensor 203.

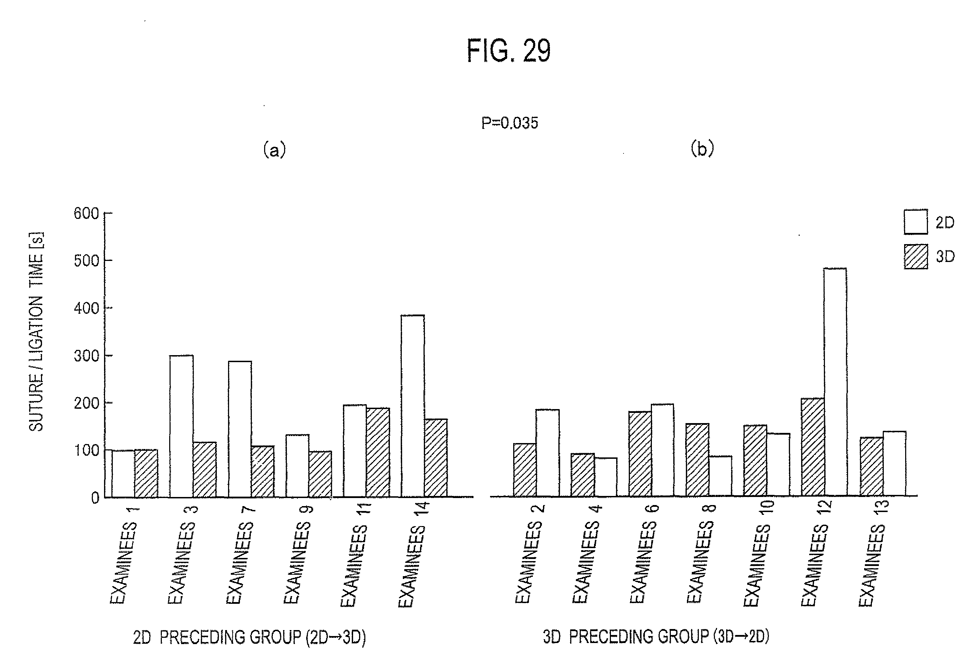

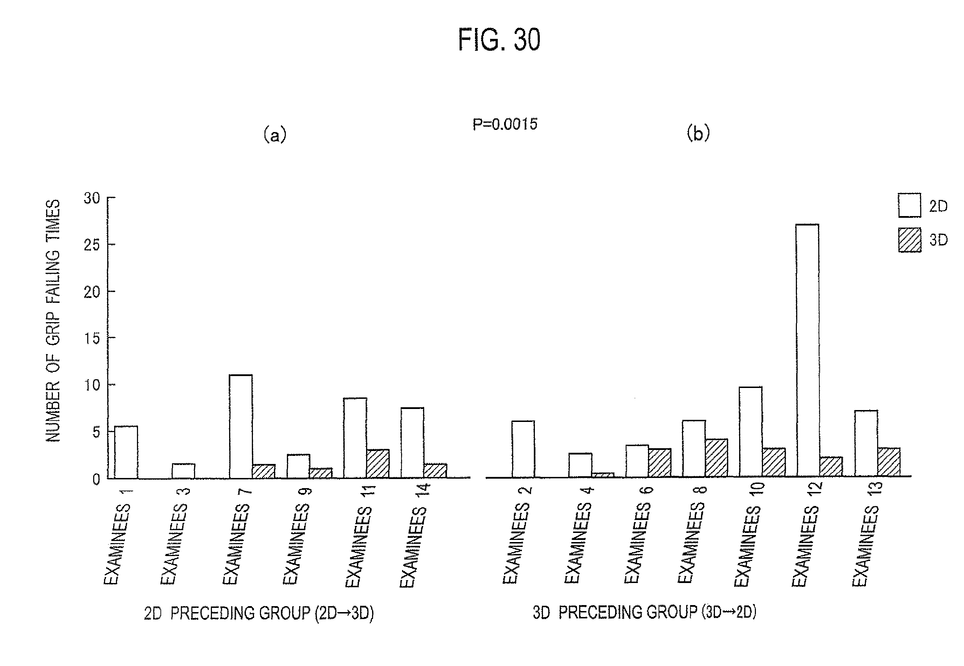

As results of the predetermined fourth operation task, FIG. 29 shows the suture/ligation time [sec] of each suture string, and FIG. 30 shows the number of grip failing times [number of times] of the suture string or a suture needle. FIG. 29A and FIG. 30A show results in the case where the fourth operation task is performed while seeing the three-dimensional video (3D) after the fourth operation task is performed while seeing the two-dimensional video (2D). FIG. 29B and FIG. 30B show results in the case where the fourth operation task is performed while seeing the two-dimensional video (2D) after the fourth operation task is performed while seeing the three-dimensional video (3D). Note that the training results were acquired in such a manner that the plurality of operators P performed the fourth operation task.

As apparent from FIG. 29, in the case of the display by the three-dimensional video (3D), the suture/ligation time of the suture string is shorter than in the case of the display by the two-dimensional video (2D). Note that the value of P is also 0.03. Moreover, as apparent from FIG. 30, in the case of the display by the three-dimensional video (3D), the number of grip failing time is smaller than in the case of the display by the two-dimensional video (2D). Note that the value of P is also 0.0015.

[First Recognition Task]

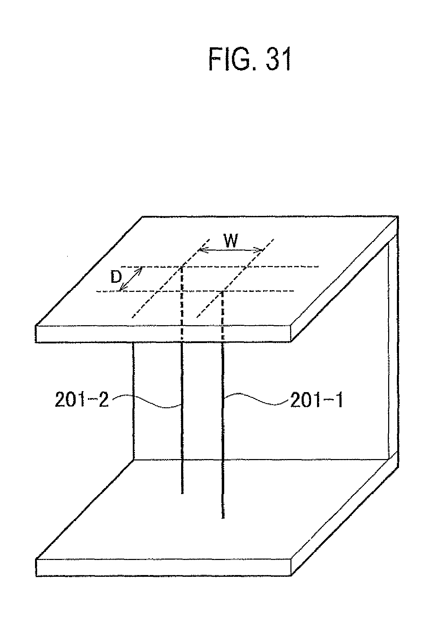

For the first recognition task, a simulation sample as shown in FIG. 31 was used. The first recognition task is training for accurately grasping the front and rear positional relationship between a plurality of stringy targets 201-1 and 201-2 in which distances from the imaging position of the endoscope device 2 differ from each other by D.

Training results of the first recognition task are obtained for both of the case where the two-dimensional video (2D) is displayed on the flat monitor and the case where the three-dimensional video (3D) is displayed on the above-mentioned dome type screen 11. Moreover, the training results of the first recognition task are obtained for the case (3DP) where the first recognition task is performed while displaying the stereoscopic video by using a monitor in which the display screen is flat and for the case (3DD) where the first recognition task is performed while displaying the stereoscopic video on the above-mentioned dome type screen 11. Note that, in all of the cases, the same simulation sample and the same endoscopic operation-use display apparatus 1 were used.

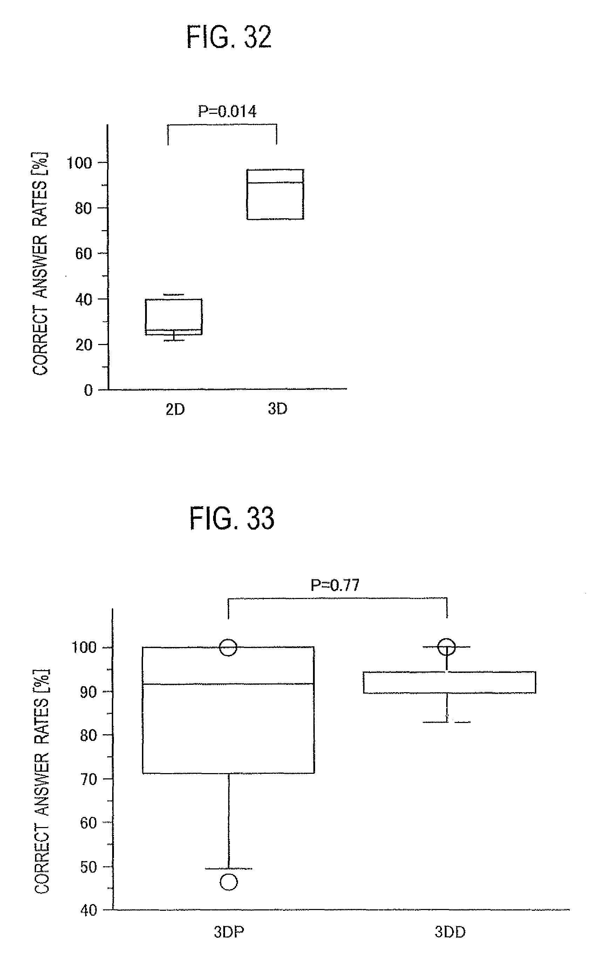

As results of the first recognition task, FIG. 32 shows correct answer rates of the front and rear positional relationship in the case where the two-dimensional video (2D) is used and the case where the three-dimensional video (3D) is used. Moreover, FIG. 33 shows correct answer rates of the front and rear positional relationship in the case (3DP) where the three-dimensional video is displayed on the flat monitor and the case (3DD) where the three-dimensional video is displayed on the dome type screen 11. Note that the training results were acquired in such a manner that the plurality of operators P performed the first recognition task.

When FIG. 32 is viewed, a remarkably large difference in correct answer rate can be confirmed between the case where the two-dimensional video is used and the case where the three-dimensional video is used. Moreover, the value of P is also 0.014. Meanwhile, when FIG. 33 is viewed, variations in correct answer rate among the examinees are large in 3DP using the flat monitor, whereas variations in correct answer rate among the examinees are small in 3DD using the dome type screen 11. Moreover, in 3DD, there is no mild outlier, and the minimum value thereof is high. Note that the value of P is 0.77.

[Second Recognition Task]

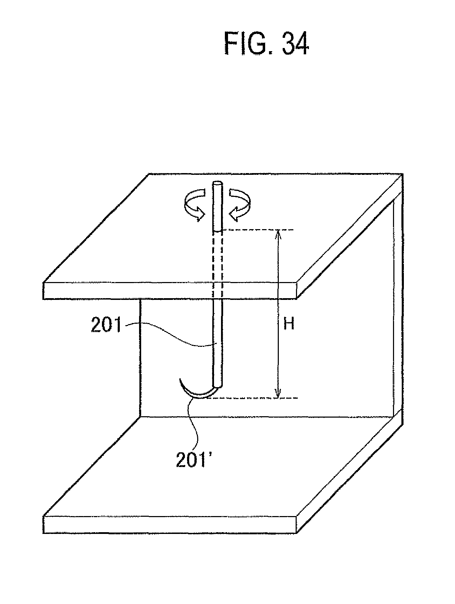

For the second recognition task, a simulation sample as shown in FIG. 34 was used. This second recognition task is training for accurately grasping an orientation of a needle-like portion 201' of a target 201 with respect to the operators P.

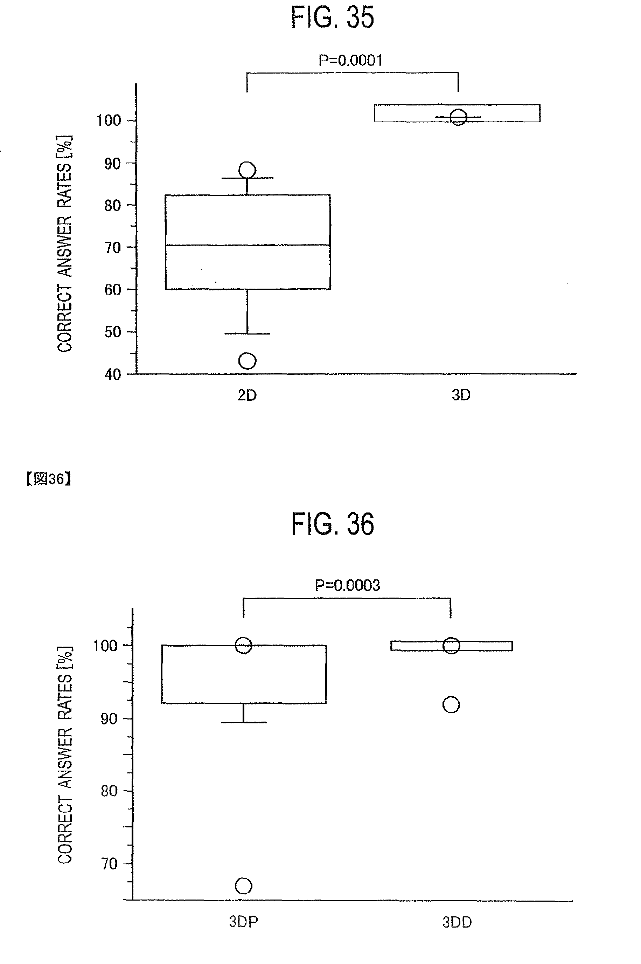

As results of this second recognition task, FIG. 35 shows correct answer rates of the orientation of the needle-like portion 201' in the case where the two-dimensional video (2D) is used and the case where the three-dimensional video (3D) is used by the above-mentioned dome type screen 11. Moreover, FIG. 36 shows correct answer rates of the orientation of the needle-like portion 201' in the case (3DP) where the three-dimensional video is displayed on the flat monitor and the case (3DD) where the three-dimensional video is displayed on the dome type screen 11. Note that the training results were acquired in such a manner that the plurality of operators P performed the second recognition task.

When FIG. 35 is viewed, a remarkably large difference in correct answer rate can be confirmed between the case where the two-dimensional video is used and the case where the three-dimensional video is used. Moreover, in the case where the three-dimensional video is used, variations in correct answer rate are concentrated to a range of 90% or more, and the variations for each of the operators P become remarkably smaller than in the case of using the two-dimensional video. Moreover, the value of P is also 0.0001, and a large significant difference is recognized there.

When FIG. 36 is viewed, variations in correct answer rate among the examinees are large in 3DP using the flat monitor, whereas variations in correct answer rate among the examinees are small in 3DD using the dome type screen 11. Moreover, in 3DD, the correct answer rate is 90% at the mild outlier, and the minimum value thereof is also extremely high. Note that the value of P is 0.0003.

In this second recognition task, even if the same stereoscopic video is displayed, a remarkably high effect is recognized by using the dome type screen 11 rather than the flat monitor.

[Third Recognition Task]

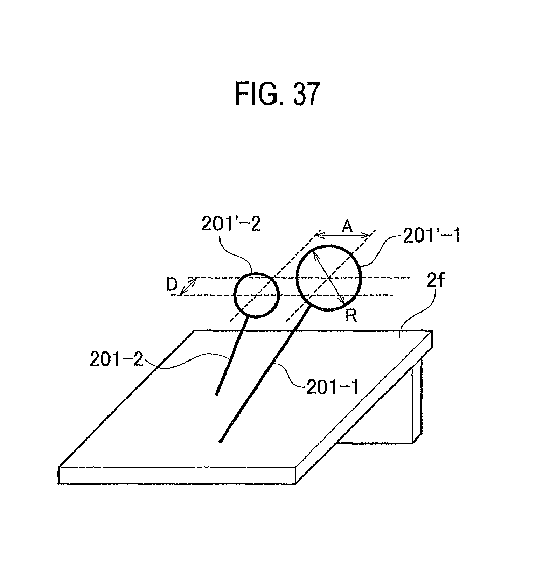

For the third recognition task, a simulation sample as shown in FIG. 37 was used. This third recognition task is training for allowing the operators P to accurately grasp a front and rear positional relationship between targets 201-1 and 201-2 in the case where the targets 201-1 and 201-2 differ from each other in distance D and width A with respect to the operators P and differ from each other in size R of annular portions 201'.

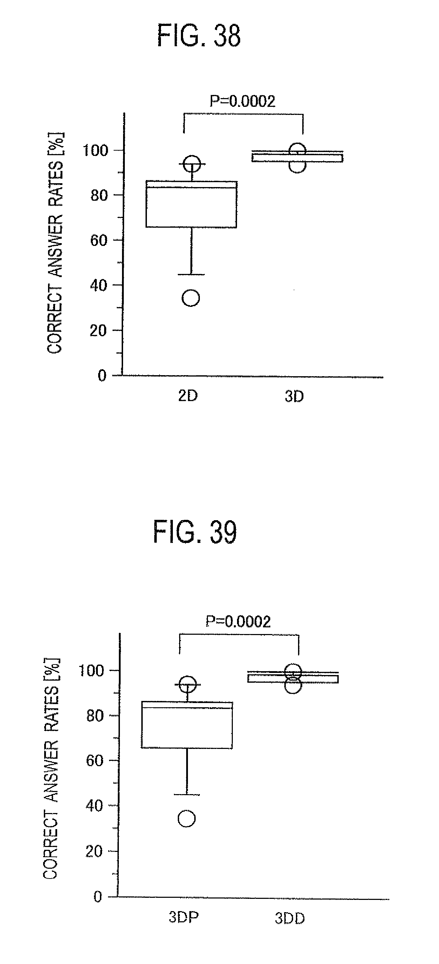

As results of this third recognition task, FIG. 38 shows correct answer rates of the front and rear positional relationship between the annular portions 201' in the case where the two-dimensional video (2D) is used and the case where the three-dimensional video (3D) is used by the dome type screen 11. Moreover, FIG. 39 shows results of the correct answer rates in 3DP where the flat monitor is used and 3DD where the dome type screen 11 is used.

When FIG. 38 is viewed, a difference in correct answer rate can be confirmed between the case where the two-dimensional video is used and the case where the three-dimensional video is used. Moreover, in the case where the three-dimensional video is used, variations in correct answer rate are concentrated to a range of 90% or more, and the variations for each of the operators P become remarkably smaller than in the case of using the two-dimensional video. Moreover, the value of P is also 0.0002, and a large significant difference is recognized there.

When FIG. 39 is viewed, the variations in 3DD using the dome type screen 11 are smaller than in 3DP using the flat monitor, and a mean value of the correct answer rates is also high. Note that the value of P is 0.0002, and a remarkable difference is recognized between the results 3DP and the results in 3DD in the third recognition task.

In this third recognition task, even if the same stereoscopic video is displayed, a remarkably high effect is recognized by using the dome type screen 11 rather than the flat monitor.

Note that the above-described embodiment is merely an example of the present invention. Therefore, the present invention is not limited to the above-mentioned embodiment, and it is a matter of course that a variety of modifications from this embodiment are possible in response to design and the like without departing from the technical spirit according to the present invention.

INDUSTRIAL APPLICABILITY

In accordance with the present invention, the video displaying means is formed into such a shape that enables the observation of the whole of the video from the first viewpoint and the observation of the center of the projection surface from the second viewpoint positions, or that enables the observation of the whole of the video from both of the first viewpoint position and the second viewpoint positions. Accordingly, the operator at the first viewpoint position and the persons at the second viewpoint positions can be allowed to always see the clear stereoscopic video.

**Claims**

1. An endoscope system that acquires a video of an imaging target in a patient's body cavity at an operation site where an operation tool inserted into the patient's body cavity is manipulated by a first operator located at a first viewpoint position, comprising:

an endoscope device that is manipulated by a second operator located at a second viewpoint position, and takes a video of a patient's affected area by allowing at least a part thereof to be inserted into the patient's body cavity;

video projecting means for projecting video light indicating the video taken by the endoscope device;
video displaying means having a shape of a projection surface that directs a concave surface toward the first operator and the second operator, in which the video light is projected onto the projection surface by the video projecting means; and
video signal processing means for performing, based on a positional relationship between at least the first viewpoint position and the video displaying means and on the shape of the projection surface, distortion correction processing for a video signal inputted to the video projecting means so that the video can be displayed without distortion on the projection surface when seen from the first viewpoint position,
wherein, in a case where an axis that passes through a center of an opening surface serving as the concave surface and is perpendicular to the opening surface is defined as a first axis, a point where the first axis and the projection surface intersect each other is defined as a projection surface center, and an axis that connects the projection surface center and an edge portion of the projection surface to each other is defined as a second axis, and a tangential line of the edge portion of the projection surface composed of a part of a spherical shape of the video displaying means is defined as a third axis, then an angle made by the first axis and the third axis is an angle at which it is possible to observe a whole of the video from the first viewpoint position, and an angle made by the first axis and the second axis is an axis at which it is possible to observe the projection surface center from the second viewpoint position.

2. An endoscope system that acquires a video of an imaging target in a patient's body cavity at an operation site where an operation tool inserted into the patient's body cavity is manipulated by a first operator located at a first viewpoint position, comprising:

an endoscope device that is manipulated by a second operator located at a second viewpoint position, and takes a video of a patient's affected area by allowing at least a part thereof to be inserted into the patient's body cavity;
video projecting means for projecting video light indicating the video taken by the endoscope device;
video displaying means having a shape of a projection surface that directs a concave surface toward the first operator and the second operator, in which the video light is projected onto the projection surface by the video projecting means; and
video signal processing means for performing, based on a positional relationship between at least the first viewpoint position and the video displaying means and on the shape of the projection surface, distortion correction processing for a video signal inputted to the video projecting means so that the video can be displayed without distortion on the projection surface when seen from the first viewpoint position,
wherein, in a case where an axis that passes through a center of an opening surface serving as the concave surface and is perpendicular to the opening surface is defined as a first axis, a point where the first axis and the projection surface intersect each other is defined as a projection surface center, and an axis that connects the projection surface center and an edge portion of the projection surface to each other is defined as a second axis, and a tangential line of the edge portion of the projection surface composed of a part of a spherical shape of the video displaying means is defined as a third axis, then an angle made by the first axis and the third axis is an angle at which it is possible to observe a whole of the video from both of the first viewpoint position and the second viewpoint position.

3. The endoscope system according to either one of claims 1 and 2, wherein, based on a relative positional relationship among the video projecting means, the first viewpoint position and the video displaying means and on the shape of the projection surface, the video signal processing means performs the distortion correction processing for the video signal inputted to the video projecting means so that the video can be displayed without distortion on the projection surface when seen from the first viewpoint position.

4. The endoscope system according to any one of claims 1 to 3, wherein the video displaying means is configured so that the angle made by the first axis and the second axis can be set within a range from 11 degrees to 82 degrees.

5. The endoscope system according to any one of claims 1 to 3, wherein the video displaying means is configured so that the angle made by the first axis and the third axis can be set within a range from 11 degrees to 73 degrees.

6. The endoscope system according to any one of claims 1 to 5, wherein, by using the video signal acquired by the endoscope device, the video projecting means projects video light indicating a stereoscopic video visually recognizable by stereoscopic glasses worn by the first operator and the second operator.

7. The endoscope system according to any one of claims 1 to 6, further comprising: a light source unit including at least a light source and a filter, which make a tissue state of the affected area identifiable by emission of irradiation light onto a video imaging range.

8. The endoscope system according to any one of claims 1 to 7, further comprising: a living body information acquisition unit that acquires living body information of the patient, wherein the video projecting means projects video light in which the living body information acquired by the living body information acquisition unit and a medical image acquired before and during an operation are superposed on the video of the affected area of the patient.

9. The endoscope system according to any one of claims 1 to 8, wherein the video projecting means is made capable of switching between a non-stereoscopic video signal and a stereoscopic video signal, which are acquired by the endoscope device, and allows the video displaying means to display the non-stereoscopic video or the stereoscopic video thereon.

10. The endoscope system according to any one of claims 1 to 9, further comprising:

    a base portion that supports a single mobile body formed by assembling the video projecting means and the video displaying means integrally with each other; and
    lifting means for moving the mobile body vertically with respect to the base portion.

11. The endoscope system according to claim 10, further comprising: a reflecting mirror that reflects the video light projected from the video projecting means and guides the video light to the projection surface of the video displaying means, wherein the base portion supports a single mobile body formed by assembling the reflecting mirror integrally with the video projecting means and the video displaying means.

12. The endoscope system according to either one of claims 10 and 11, further comprising: rotating means for rotating the mobile body about a perpendicular shaft that moves the mobile body vertically.

13. The endoscope system according to any one of claims 10 to 12, wherein the base portion includes a caster mechanism that moves the mobile body.

14. An endoscopic operation training system that performs training of an endoscopic operation, comprising:

    a simulation sample that briefly simulates an affected area of a patient as an operation target, and receives insertion of an operation tool manipulated by a first operator located at a first viewpoint position;
    an endoscope device that is manipulated by a second operator located at a second viewpoint position, and takes a video of the affected area of the patient by allowing at least a part thereof to be inserted into a patient's body cavity;
    video projecting means for projecting video light indicating the video taken by the endoscope device;
    video displaying means having a shape of a projection surface that directs a concave surface toward the first operator and the second operator, in which the video light is projected onto the projection surface by the video projecting means; and
    video signal processing means for performing, based on a positional relationship between at least the first viewpoint position and the video displaying means and on the shape of the projection surface, distortion correction processing for a video signal inputted to the video projecting means so that the video can be displayed without distortion on the projection surface when seen from the first viewpoint position,
    wherein, in the video displaying means, in a case where an axis that passes through a center of an opening surface serving as the concave surface and is perpendicular to the opening surface is defined as a first axis, a point where the first axis and the projection surface intersect each other is defined as a projection surface center, and an axis that connects the projection surface center and an edge portion of the projection surface to each other is defined as a second axis, and a tangential line of the edge portion of the projection surface composed of a part of a spherical shape of the video displaying means is defined as a third axis, then an angle made by the first axis and the third axis is an angle at which it is possible to observe a whole of the video from the first viewpoint position, and an angle made by the first axis and the second axis is an axis at which it is possible to observe the projection surface center from the second viewpoint position.

15. An endoscopic operation training system that acquires a video of an imaging target in a patient's body cavity at an operation site where an operation tool inserted into the patient's body cavity is manipulated by a first operator located at a first viewpoint position, comprising:

an endoscope device that is manipulated by a second operator located at a second viewpoint position, and takes a video of an affected area of a patient by allowing at least a part thereof to be inserted into the patient's body cavity;

video projecting means for projecting video light indicating the video taken by the endoscope device;

video displaying means having a shape of a projection surface that directs a concave surface toward the first operator and the second operator, in which the video light is projected onto the projection surface by the video projecting means; and

video signal processing means for performing, based on a positional relationship between at least the first viewpoint position and the video displaying means and on the shape of the projection surface, distortion correction processing for a video signal inputted to the video projecting means so that the video can be displayed without distortion on the projection surface when seen from the first viewpoint position,

wherein, in the video displaying means, in a case where an axis that passes through a center of an opening surface serving as the concave surface and is perpendicular to the opening surface is defined as a first axis, a point where the first axis and the projection surface intersect each other is defined as a projection surface center, and an axis that connects the projection surface center and an edge portion of the projection surface to each other is defined as a second axis, and a tangential line of the edge portion of the projection surface composed of a part of a spherical shape of the video displaying means is defined as a third axis, then an angle made by the first axis and the third axis is an angle at which it is possible to observe a whole of the video from both of the first viewpoint position and the second viewpoint position.

16. The endoscopic operation training system according to either one of claims 14 and 15, wherein, based on a relative positional relationship among the video projecting means, the first viewpoint position and the video displaying means and on the shape of the projection surface, the video signal processing means performs the distortion correction processing for the video signal so that the video can be displayed without distortion on the projection surface when seen from the first viewpoint position.

# FIG. 1

TO ENDOSCOPE DEVICE

# FIG. 2

# FIG. 3

```
┌─────────────────────────┐
│     ENDOSCOPE DEVICE    │────2
└─────────────────────────┘
            │
            ▼
┌───────────────────────────────────────────────────────┐
│ VIDEO SIGNAL PROCESSING DEVICE                    ──3  │
│                                                         │
│   3B          ┌─────────────────────┐                  │
│               │     CONTROL UNIT    │────3A            │
│               └─────────────────────┘                  │
│  ┌──────────────────┐  ┌──────────────────────┐        │
│  │ LEFT EYE-USE VIDEO│  │ RIGHT EYE-USE VIDEO  │──3C    │
│  │ CORRECTION UNIT   │  │ CORRECTION UNIT      │        │
│  └──────────────────┘  └──────────────────────┘        │
│                        ┌──────────────────────┐        │
│                        │ DISTORTION CORRECTION│──3D    │
│                        │ TABLE STORAGE UNIT   │        │
│                        └──────────────────────┘        │
└───────────────────────────────────────────────────────┘
            │
            ▼
┌─────────────────────────┐
│       PROJECTORS        │──12,13
└─────────────────────────┘
            ┊──── STEREOSCOPIC VIDEOS
            ▼
┌─────────────────────────┐
│    POLARIZATION FILTER  │──12b,13b
└─────────────────────────┘
            ┊
            ▼
┌─────────────────────────┐
│    REFLECTING MIRROR    │──14
└─────────────────────────┘
            ┊
            ▼
┌─────────────────────────┐
│     DOME TYPE SCREEN    │──11
└─────────────────────────┘
            ┊
            ▼
┌─────────────────────────┐
│   STEREOSCOPIC GLASSES  │──5
└─────────────────────────┘
```

FIG. 4

FIG. 5

TO ENDOSCOPE DEVICE

FIG. 6

FIG. 7

FIG. 8

FIG. 9

# FIG. 10

## FIG. 11

LOGISTICS

ENDOSCOPIC
OPERATION-USE
DISPLAY
APPARATUS

1

ASSISTANT

CAMERAMAN

LOGISTICS

PEDAL

PATIENT

BED

OPERATOR

MOUNTING
(FORCEPS)

ASSISTANT

PEDAL

LOGISTICS

## FIG. 12

ASSISTANT A
(SECOND VIEWPOINT)

BED

ATTENTION
POINT

ANGLE $\gamma$

500mm

C=A+B

A

B
2000mm

E

F=D+E

D

OPERATOR
(FIRST VIEWPOINT)

ASSISTANT B (SECOND VIEWPOINT)

## FIG. 13

ASSISTANT A
(SECOND VIEWPOINT)

E | F=D+E

ATTENTION POINT

ANGLE
$\delta$

D

2000mm

A | B

OPERATOR
(FIRST VIEWPOINT)

C=A+B

500mm

ASSISTANT B
(SECOND VIEWPOINT)

## FIG. 14

P3

L2

11b

11

L1

P2

P1

d

## FIG. 15

11

P1

ANGLE α

VERTICAL AXIS

11a

## FIG. 16

L3

P3

11

β

L1

P2

11b

# FIG. 17

11

ANGLE β

11b

11a

11b

VERTICAL AXIS

# FIG. 18

FIG. 19

FIG. 20

FIG. 21

FIG. 22

FIG. 23

FIG. 24

2D
3D

P=0.018

NUMBER OF GRIP FAILING TIMES

EXAMINEES 1　EXAMINEES 2　EXAMINEES 3　EXAMINEES 4　EXAMINEES 5　EXAMINEES 6　EXAMINEES 7　EXAMINEES 8　EXAMINEES 9

# FIG. 25

201-2

201-1

203

204

# FIG. 26

# FIG. 27

FIG. 28

# FIG. 29

P=0.035

(a)

(b)

SUTURE / LIGATION TIME [s]

2D

3D

EXAMINEES 1
EXAMINEES 3
EXAMINEES 7
EXAMINEES 9
EXAMINEES 11
EXAMINEES 14

2D PRECEDING GROUP (2D→3D)

EXAMINEES 2
EXAMINEES 4
EXAMINEES 6
EXAMINEES 8
EXAMINEES 10
EXAMINEES 12
EXAMINEES 13

3D PRECEDING GROUP (3D→2D)

## FIG. 30

P=0.0015

(a)                                    (b)

2D PRECEDING GROUP (2D→3D)          3D PRECEDING GROUP (3D→2D)

EP 2 260 753 A1

FIG. 31

# FIG. 32

P=0.014

# FIG. 33

P=0.77

FIG. 34

# FIG. 35

【図36】

# FIG. 36

# FIG. 37

## FIG. 38

## FIG. 39

<table>
<tr><td colspan="2" align="center"><strong>INTERNATIONAL SEARCH REPORT</strong></td><td colspan="2">International application No.<br><br>PCT/JP2008/068973</td></tr>
</table>

| A. CLASSIFICATION OF SUBJECT MATTER |
|---|
| *A61B1/04*(2006.01)i, *G03B21/10*(2006.01)i |

According to International Patent Classification (IPC) or to both national classification and IPC

| B. FIELDS SEARCHED |
|---|

Minimum documentation searched (classification system followed by classification symbols)
A61B1/04, G03B21/10

Documentation searched other than minimum documentation to the extent that such documents are included in the fields searched

| Jitsuyo Shinan Koho | 1922-1996 | Jitsuyo Shinan Toroku Koho | 1996-2009 |
|---|---|---|---|
| Kokai Jitsuyo Shinan Koho | 1971-2009 | Toroku Jitsuyo Shinan Koho | 1994-2009 |

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| X<br>Y | JP 2008-15470 A  (Matsushita Electric Works, Ltd.),<br>24 January, 2008 (24.01.08),<br>Par. Nos. [0005], [0007], [0012] to [0013], [0016], [0026], [0039] to [0042]; Figs. 1 to 3<br>& WO 2007/141933 A1 | 1-3,6,10-13<br>4-5,14-16 |
| Y | JP 2003-522977 A  (Elumens Corp.),<br>29 July, 2003 (29.07.03),<br>Par. No. [0019]; Fig. 7<br>& EP 1256107 A1        & WO 2001/059738 A1 | 4-5 |
| Y | JP 2000-512027 A  (Simulab Corp.),<br>12 September, 2000 (12.09.00),<br>Page 6, lines 17 to 19<br>& EP 902936 A1          & WO 1997/044768 A1 | 14-16 |

☐  Further documents are listed in the continuation of Box C.    ☐  See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    13 January, 2009 (13.01.09) | Date of mailing of the international search report<br>    27 January, 2009 (27.01.09) |
|---|---|
| Name and mailing address of the ISA/<br>    Japanese Patent Office | Authorized officer |
| Facsimile No. | Telephone No. |

Form PCT/ISA/210 (second sheet) (April 2007)

## INTERNATIONAL SEARCH REPORT

| International application No. |
|---|
| PCT/JP2008/068973 |

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:
    JP 2008-15470 A (hereinafter referred to as "document 1"), paragraphs [0005], [0007], [0012]-[0013], [0016], [0026] and [0039]-[0042], Figs. 1-3, describes that a video image display device for an endoscope is provided with a video signal processing unit (1) to carry out distortion correction processing, projectors (20, 21), a reflecting mirror (4) and a semi-spherical screen (5).

    With reference to Fig. 2 of the document 1, it is understood that the entire video image can be observed from a position (corresponding to the first viewing

                                    (To be continued on the extra sheet)

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.   Consequently, this international search report is restricted to the invention first mentioned in the claims; it is covered by claims Nos.:

| **Remark on Protest** | ☐ The additional search fees were accompanied by the applicant's protest and, where applicable, payment of a protest fee. |
|---|---|
| the | ☐ The additional search fees were accompanied by the applicant's protest but the applicable protest fee was not paid within the time limit specified in the invitation. |
| | ☐ No protest accompanied the payment of additional search fees. |

Form PCT/ISA/210 (continuation of first sheet (2)) (April 2007)

**INTERNATIONAL SEARCH REPORT**

International application No.

PCT/JP2008/068973

Continuation of Box No.III of continuation of first sheet(2)

point) between B and C and the center of the projecting surface can be observed from a position (corresponding to the second viewing point) of C.

Thus, the feature specified in claim 1 common to each invention is not deemed to be "the special technical feature" in the meaning of PCT Rule 13.2, second sentence.

With this recognition, "the special technical feature" cannot be said unless an angle that makes it possible to observe the entire video image and an angle that makes it possible to observe the center of the projecting surface are set to be 11° through 82° and 11° through 73° in the inventions in claims 4-5, respectively.

Contrary to this, since the technical feature of the invention in claim 7 is the structure of a light source unit including a light source that makes it possible to identify a tissue condition of the diseased part and a filter, claim 7 cannot have a technical relationship with claims 4-5.

The same analysis of the claims as set forth above is made as follows:

The invention in claim 8 has such a technical feature that biological information and a medical image are overlapped to be projected.

The invention in claim 9 has a technical feature that makes a non-three-dimensional video signal and a three-dimensional video signal switchable.

Therefore, the inventions in claims 1-16 do not comply with the requirement of unity of invention, and the special technical features are categorized into the following four:

Claims 1-6 and 10-16: a screen shape
Claim 7: a light source unit including a filter
Claim 8: an overlapped image
Claim 9: switching of a plane/three-dimensional image

Form PCT/ISA/210 (extra sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008015470 A